# EUROPEAN PATENT APPLICATION

(11) **EP 4 847 018 A1**
(43) Date of publication of application: **02.12.2026**
(21) Application number: 25305781.4
(22) Date of filing: 30.05.2025
(51) Int. Cl.: A61K 31/165, A61K 31/4178, A61P 1/16

(54) **COMPOUNDS FOR USE IN THE PREVENTION AND/OR TREATMENT OF CHOLESTATIC LIVER DISEASES**

(71) Applicant: SORBONNE UNIVERSITE, 75006 Paris (FR); Institut National de la Santé et de la Recherche Médicale, 75013 Paris (FR); Assistance Publique - Hôpitaux de Paris, 75012 Paris (FR)
(72) Inventor: GAUTHERON, Jérémie, 75012 Paris (FR); SORET, Pierre-Antoine, 75012 Paris (FR); RATZIU, Vlad, 75013 Paris (FR)
(74) Representative: Gevers & Orès

(57) **Abstract**

The present invention relates to compounds and compositions comprising said compounds, for use in the prevention and/or treatment of a cholestatic liver disease.

## Description

The present invention relates to compounds and compositions comprising said compounds, for use in the prevention and/or treatment of cholestatic liver diseases.

Cholestatic liver diseases encompass a group of chronic and acute conditions characterized by impaired bile flow, leading to the accumulation of bile acids in the liver. This accumulation can result in hepatocellular damages, fibrosis, and eventually cirrhosis. Patients often experience symptoms such as jaundice, fatigue, and pruritus, which can significantly affect their quality of life.

Primary sclerosing cholangitis (PSC) (formerly known as primary biliary cirrhosis) is a progressive cholestatic liver disease of unknown etiology, characterized by inflammation, fibrosis, and stricturing of intrahepatic and extrahepatic bile ducts. PSC is often associated with severe complications, including biliary cirrhosis, portal hypertension, and an increased risk of cholangiocarcinoma. Fibrosis, which is marked by the excessive deposition of extracellular matrix (ECM) proteins, is a central feature of PSC and a major driver of disease morbidity and mortality. It arises from chronic biliary inflammation and persistent injury to cholangiocytes, which are the epithelial cells lining the bile ducts. In response to sustained damage, hepatic stellate cells (HSCs) and other ECM-producing cells are activated, undergoing myofibroblastic transformation with secretion of collagen and other ECM components. This pathological ECM accumulation leads to tissue stiffening, bile duct scarring, and progressive loss of liver function. Similar to other chronic liver diseases, fibrosis in PSC is a dynamic and potentially reversible process, however the precise cellular and molecular pathways regulating this response are still poorly defined.

Despite ongoing research, the precise pathophysiological mechanisms underlying PSC remain poorly understood, and no effective disease-modifying treatments currently exist. Due to this, PSC is often referred to as "the last black box" in hepatology, underscoring the significant gaps in the understanding of the disease and the urgent need for novel therapeutic strategies. The only curative option remains liver transplantation, which is associated with high morbidity and mortality, and up to 30 % of transplanted PSC patients experience disease recurrence.

There thus remains a genuine need for an effective prevention or treatment of cholestatic liver diseases.

The present invention is believed to meet such need by providing compounds and compositions for preventing and/or treating cholestatic liver diseases.

Surprisingly, the Inventors have observed that receptor-interacting serine/threonine-protein kinase 1 (RIPK-1) inhibitors have positive effects to reduce cholestatic liver diseases.

Receptor-interacting serine/threonine-protein protein kinase (RIPK)-1, along with RIPK3 and the mixed lineage kinase domain-like pseudokinase (MLKL), mediates necroptosis, a regulated process which can exacerbate inflammation and tissue damage. Recent studies suggest that RIPK-1 acts as a critical regulator of cell fate by modulating the balance between cell survival through the nuclear factor-κB (NF-κB) pathway, apoptosis, and necroptosis. Given its role in controlling multiple cell death pathways, RIPK-1 has emerged as a potential therapeutic target in various diseases. Preclinical studies have shown that RIPK-1 inhibition can reduce cell death and inflammation in models of ischemia-reperfusion injury (IRI) in the heart (Smith et al., Cardiovasc Drugs Ther 21, 227-233, 2007), liver (Shi et al., Liver Transpl 25, 1091-1104, 2019) and kidney (Jouan-Lanhouet et al., Semin Cell Dev Biol 35, 2-13, 2014), as metabolic dysfunction-associated steatotic liver disease (MASLD) (Majdi et al., J Hepatol 72, 627-635, 2020; WO 2021078958), and in neurodegenerative disease models (Ofengeim et al., Cell Rep 10, 1836-1849, 2015), largely by disrupting necroptosis and limiting pro-inflammatory signaling cascades.

The Inventors investigated the role of cell death in cholestatic liver diseases and leveraged CRISPR/Cas9-based genetic ablation strategies in cholangiocytes to dissect the molecular underpinnings of necroptotic and apoptotic signaling. Building on these mechanistic insights, the therapeutic potential of RIPK-1 inhibition in a preclinical model of PSC was assessed, focusing on its impact on biliary fibrosis and disease progression. Unexpectedly, the experimental results support a novel therapeutic strategy targeting cell death pathways to alleviate fibrosis and improve clinical outcomes in cholestatic liver diseases, in particular in PSC.

In this context, the present invention concerns a receptor-interacting RIPK-1 inhibitor or a composition comprising the same for use in the prevention and/or treatment of a cholestatic liver disease.

The present invention further relates to a method for preventing and/or treating a cholestatic liver disease by means of administration, to a patient in need thereof, of an effective amount of a RIPK-1 inhibitor or a composition comprising the same.

The present invention further relates to the use of a RIPK-1 inhibitor or a composition comprising the same for the manufacture of a medication for preventing and/or treating a cholestatic liver disease.

In the context of the present invention, the expression "receptor-interacting serine/threonine kinase 1" or "RIPK-1" pertains to a protein according to the amino acid sequence shown in **SEQ ID NO: 1.** RIPK-1 is also known as "receptor (TNFRSF)-interacting serine-threonine kinase 1" or "receptor-interacting protein kinase 1" (RIP) (HUGO Gene Nomenclature Committee symbol: HGNC:10019, database version of May 2025). The human *RIPK-1* gene is located 6p25.2, homologs are known from mouse (MGL108212; NCBI Gene: 19766) and rat (Rat Genome Database (RGD) ID: 1310158).

**Table 1. Amino acid sequence of human RIPK-1.**

| | |
|---|---|
| **SEQ ID NO: 1** | MQPDMSLNVIKMKSSDFLESAELDSGGFGKVSLCFHRTQGLMIMKTVYKGPNCIEH NEALLEEAKMMNRLRHSRVVKLLGVIIEEGKYSLVMEYMEKGNLMHVLKAEMSTPLS VKGRIILEIIEGMCYLHGKGVIHKDLKPENILVDNDFHIKIADLGLASFKMWSKLNNEEH NELREVDGTAKKNGGTLYYMAPEHLNDVNAKPTEKSDVYSFAVVLWAIFANKEPYEN AICEQQLIMCIKSGNRPDVDDITEYCPREIISLMKLCWEANPEARPTFPGIEEKFRPFYLS QLEESVEEDVKSLKKEYSNENAVVKRMQSLQLDCVAVPSSRSNSATEQPGSLHSSQGL GMGPVEESWFAPSLEHPQEENEPSLQSKLQDEANYHLYGSRMDRQTKQQPRQNVA YNREEERRRRVSHDPFAQQRPYENFQNTEGKGTAYSSAASHGNAVHQPSGLTSQPQ VLYQNNGLYSSHGFGTRPLDPGTAGPRVWYRPIPSHMPSLHNIPVPETNYLGNTPTM PFSSLPPTDESIKYTIYNSTGIQIGAYNYMEIGGTSSSLLDSTNTNFKEEPAAKYQAIFDN TTSLTDKHLDPIRENLGKHWKNCARKLGFTQSQIDEIDHDYERDGLKEKVYQMLQKW VMREGIKGATVGKLAQALHQCSRIDLLSSLIYVSQN |

The expressions "RIPK-1" or "RIPK-1 protein" as used in context of the herein disclosed invention shall pertain to a protein (such as a full-length protein, fusion protein or partial protein) comprising a sequence as shown in **SEQ ID NO: 1.** The terms shall also refer to a protein comprising the amino acid sequence according to SEQ ID NO: 1 with any protein modifications. Such protein modifications preferably do not alter the amino acid sequence of the polypeptide chain, but constitute a functional group, which is conjugated to the basic amino acid polymer chain. Protein modifications in context of the invention may be selected from a conjugation of additional amino acid sequences to the RIPK-1 amino acid chain, such as ubiquitination, sumoylation, methylation, or similar small protein conjugates. Other protein modifications include, but are not limited to, glycosylation, methylation, lipid-conjugation, or other natural or artificial post-translational modifications known to the skilled person.

The expressions "RIPK-1" or "RIPK-1 protein" as used in context of the herein disclosed invention shall also pertain to a variant of RIPK-1. A variant of RIPK-1 is, in some embodiments, a protein comprising an amino acid sequence having at least 60%, 70%, 80%, 90%, preferably at least 80% such as at least 90%, sequence identity to SEQ ID NO: 1, and most preferably at least 95% (such as at least 98%) sequence identity to **SEQ ID NO: 1.** In one preferred embodiment of the invention, the variant of RIPK-1 comprises an amino acid sequence with at least 80% sequence identity to the amino acid sequence shown in **SEQ ID NO: 1.**

As used herein, the terms "identical" or percent "identity", when used anywhere herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site).

In the context of the present invention, the expression "receptor-interacting serine/threonine-protein kinase 1 inhibitor" or "RIPK-1 inhibitor" or any similar expression shall encompass any compound or combination of compounds that have an activity of inhibiting or lowering the expression, function and/or stability of RIPK-1 or of a variant of RIPK-1.

In particular, the expression "RIPK-1 inhibitor" designates well-known inhibitors, which are classically divided into four categories, as detailed in Chen et al. (Front Pharmacol, vol. 13, 2022). The first category is the type I ATP-competitive kinase inhibitors that targets the area occupied by the ATP adenylate ring. The second category is type II ATP competitive kinase inhibitors targeting the DLG-out conformation of RIPK-1. The third category is type III kinase inhibitors that compete for binding to allosteric sites near ATP pockets. The fourth category is others.

In some embodiments, the invention concerns the RIPK-1 inhibitor or the composition comprising the same for use as defined above, wherein the RIPK-1 inhibitor is a type I kinase inhibitor, a type II kinase inhibitor or a type III kinase inhibitor.

Preferred RIPK-1 inhibitors of the invention are small molecular compounds.

In some embodiments, the invention concerns the RIPK-1 inhibitor or the composition comprising the same for use as defined above, wherein the RIPK-1 inhibitor is selected from the group comprising N-benzyl-N-hydroxy-2,2-dimethylbutanamide (RIPA-56), necrostatin-1 stable (5-((7-chloro-1H-indol-3-yl)methyl)-3-methyl-2,4-imidazolidinedione), necrostatin-1 (5-(1H-indol-3-ylmethyl)-3-methyl-2-thioxo-4-imidazolidinone), necrostatin-2 (5-[(7-chloro-1H-indol-3-yl)methyl]-3-methyl-2,4-imidazolidinedione), necrostatin-3 (3-phenyl-3,3a,4,5-tetrahydro-2H-benz[g]indazole, 3-phenyl-2,3,3a,4-tetrahydro[i]benzopyrano[4,3-c]pyrazole, 3-phenyl-2,3,3a,4-tetrahydro[i]benzothiopyrano[4,3-c]pyrazole, 5,5-dioxo-3-phenyl-2,3,3a,4-tetrahydro[1]benzothiopyrano[4,3-c]pyrazole]), necrostatin-4 ((S)-N-(1-[2-chloro-6-fluorophenyl]ethyl)-5-cyano-1-methyl-1H-pyrrole-2-carboxamide), necrostatin-5 (2-[[3,4,5,6,7,8-hexahydro-3-(4-methoxyphenyl)-4-oxo[1]benzothieno[2,3-d]pyrimidin-2-yl]thio]-acetonitrile), necrostatin-7 (5-((3-(4-fluorophenyl)-1H-pyrazol-4-yl)methylene)-2-imino-3-(thiazol-2-yl)thiazolidin-4-one), necrostatin-21, (S)-5-benzyl-N-(7,9difluoro-2-oxo-2,3,4,5-tetrahydro-1H-benzo[b]azepin-3-yl)1H-1,2,4-triazole-3-carboxamide (GSK3145095), DNL747 (SAR443060), (S)-N-(5-methyl-4-oxo-2,3,4,5-tetrahydrobenzo[b][1,4]oxazepin-3-yl)-4,5,6,7-tetrahydro-1H-indazole-3-carboxamide, (S)-*N*-(5-methyl-4-oxo-2,3,4,5-tetrahydrobenzo[b][1,4]oxazepin-3-yl)-4H-1,2,4-triazole-3-carboxamide, (R)-5-methyl-*N*-((S)-5-methyl-4-oxo-2,3,4,5-tetrahydrobenzo[b][1,4]oxazepin-3-yl)-4,5,6,7-tetrahydro-1H-indazole-3-carboxamide, (R)-5-methyl-N-((S)-5-methyl-4-oxo-2,3,4,5-tetrahydrobenzo[b][1,4]oxazepin-3-yl)-4,5,6,7-tetrahydro-1H-indazole-3-carboxamide, (S)-6,6-dimethyl-N-(5-methyl-4-oxo-2,3,4,5- tetrahydrobenzo[b][1,4]oxazepin-3-yl)-4,5,6,7-tetrahydro-1H-indazole-3-carboxamide, (S)-N-(5-methyl-4-oxo-2,3,4,5-tetrahydrobenzo[b][1,4]oxazepin-3-yl)-i',4',5',7'-tetrahydrospiro[cyclopropane-1,6'-indazole]-3'-carboxamide, N-((S)-5-methyl-4-oxo-2,3,4,5- tetrahydrobenzo[b][1,4]oxazepin-3-yl)-1,4,4a,5,5a,6-hexahydrocyclopropa[f]indazole-3-carboxamide, N-((S)-5-methyl-4-oxo-2,3,4,5-tetrahydrobenzo[b][1,4]oxazepin-3-yl)-3,4,5,5a,6,6a-hexahydrocyclopropa[e]indazole-1-carboxamide, (S)-5,5-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (R)-5-(tert-butyl)-N-((S)-5-methyl-4-oxo-2,3,4,5-tetrahydrobenzo[b][1,4]oxazepin-3-yl)-4,5,6,7-tetrahydro-1H-indazole-3-carboxamide, N-((S)-5-methyl-4-oxo-2,3,4,5-tetrahydrobenzo[b][1,4]oxazepin-3-yl)-5-phenyl-4,5,6,7-tetrahydro-1H-indazole-3-carboxamide, N-((S)-5-methyl-4-oxo-2,3,4,5- tetrahydrobenzo[b][1,4]oxazepin-3-yl)-5-(1H-pyrazol-1-yl)-4,5,6,7-tetrahydro-1H-indazole-3- carboxamide, (R)-1-methyl-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (S)-N-(5-methyl-4-oxo-2,3,4,5-tetrahydrobenzo[b][1,4]oxazepin-3-yl)-5-(trifluoromethyl)-1H-indazole-3-carboxamide, (S)-N-(5-methyl-4-oxo-2,3,4,5-tetrahydrobenzo[b][1,4]oxazepin-3-yl)-5-(trifluoromethyl)-1H-pyrazolo[3,4-b]pyridine-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-[1,2,3]triazolo[1,5-a]pyridine-3-carboxamide, (S)-N-(5-methyl-4-oxo-2,3,4,5-tetrahydrobenzo[b][1,4]oxazepin-3-yl)-7-(trifluoromethyl)imidazo[1,5-a]pyridine-1-carboxamide, (S)-N-(5-methyl-4-oxo-2,3,4,5-tetrahydrobenzo[b][1,4]oxazepin-3-yl)-6-(trifluoromethyl)-[1,2,4]triazolo[4,3-b]pyridazine-3-carboxamide, (S)-5-isopropyl-N-(5-methyl-4-oxo-2,3,4,5-tetrahydrobenzo[b][1,4]oxazepin-3-yl)-1H-pyrazolo[3,4-b]pyridine-3-carboxamide, (S)-N-(5-methyl-4-oxo-2,3,4,5-tetrahydrobenzo[b][1,4]oxazepin-3-yl)-5-(perfluoroethyl)-1H-pyrazolo[3,4-b]pyridine-3-carboxamide, N-((S)-5-methyl-4-oxo-2,3,4,5-tetrahydrobenzo[b][1,4]oxazepin-3-yl)-5-(perfluoroethyl)-4,5,6,7-tetrahydro-[1,2,3]triazolo[1,5- a]pyridine-3-carboxamide, (S)-N-(5-methyl-4-oxo-2,3,4,5-tetrahydrobenzo[b][1,4]oxazepin-3-yl)-6-(perfluoroethyl)-[1,2,4]triazolo[4,3-b]pyridazine-3-carboxamide, (S)-5-benzyl-N-(5-methyl-4-oxo-2,3,4,5-tetrahydrobenzo[b][1,4]oxazepin-3-yl)isoxazole-3-carboxamide (GSK481), 1-[(55)-4,5-Dihydro-5-phenyl-1H-pyrazol-1-yl]-2,2-dimethyl-1-propanone, 2,2-Dimethyl-1-(5(S)-phenyl-4,5-dihydro-pyrazol-1-yl)-propan-1-one (GSK963), (S)-5-benzyl-N-(5-methyl-4-oxo-2,3,4,5-tetrahydrobenzo[b][1,4]oxazepin-3-yl)-1H-1,2,4-triazole-3-carboxamide (GSK2982772), 5-(indol-3-ylmethyl)-3-methyl-2-thio-hydantoin), and their derivatives, analogues or salts.

A compound of the invention according to the invention can exist in unsolvated forms as well as solvated forms, including hydrated forms. In general, the solvated forms are equivalent to unsolvated forms and are intended to be encompassed within the scope of the invention. In general, all physical forms are equivalent for the uses contemplated by the invention and are intended to be within the scope of the invention.

In some embodiments, the invention concerns the RIPK-1 inhibitor or the composition comprising the same for use as defined above, wherein the RIPK-1 inhibitor is selected from the group comprising RIPA-56, necrostatin-1 stable and necrostatin-1, in particular RIPA-56.

In some embodiments, the compound according to the invention is a selective inhibitor of receptor-interacting protein kinase 1 (RIPK-1). The selective inhibition of RIPK-1 can be demonstrated via binding assays.

By "treatment" is meant, according to the present invention, the decrease or the disappearance a disease, a disorder or one or more signs and/or symptoms. In particular, the treatment of a liver cholestatic disease can correspond to a reduction or a suppression of hepatic damages, abnormal accumulation of bile in the liver, increase in bile flow and/or fibrosis of liver.

By "prevention" is meant, according to the invention, the prevention of the appearance of a disease, a disorder or one or more signs and/or symptoms. In particular, the prevention of a liver cholestatic disease can correspond to the prevention of hepatic damages, abnormal accumulation of bile in the liver, increase in bile flow and/or fibrosis of liver.

The prevention and treatment according to the invention apply to humans and animals, preferably humans.

In some embodiments, the invention concerns the RIPK-1 inhibitor or the composition comprising the same for use as defined above, wherein the cholestatic liver disease is a chronic cholestatic liver disease.

In some embodiments, the invention concerns the RIPK-1 inhibitor or the composition comprising the same for use as defined above, wherein the chronic cholestatic liver disease is a cholangitis, preferably a fibrosing cholangitis.

In some embodiments, the invention concerns the RIPK-1 inhibitor or the composition comprising the same for use as defined above, wherein the cholangitis is a fibrosing cholangitis selected from the group comprising primary sclerosing cholangitis (PSC), primary biliary cholangitis (PBC), IgG4-related cholangitis (IRC), secondary cholangitis induced by immune checkpoint inhibitors, ischemic cholangitis, drug-induced cholangitis (e.g. ketamine), Alagille syndrome, biliary atresia and genetic cholangitis such as Progressive Familial Intrahepatic Cholestasis (PFIC), preferably PSC.

In some embodiments, the invention concerns the RIPK-1 inhibitor or the composition comprising the same for use as defined above, wherein the RIPK-1 inhibitor or the composition comprising the same is administered by enteral or parenteral route.

In some embodiments, the invention concerns the RIPK-1 inhibitor or the composition comprising the same for use as defined above, wherein the RIPK-1 inhibitor or the composition comprising the same is in the form of a solution, a food, a beverage, a pill, a tablet, a syrup, a patch, a gum, a cream, a gel, a lotion, an ointment, a powder, a capsule, a vial or a suppository.

In some embodiments, the invention concerns the RIPK-1 inhibitor or the composition comprising the same for use as defined above, wherein the RIPK-1 inhibitor or the composition comprising the same is formulated into unit dose forms from 1 to 1 000 mg, in particular from 50 to 500 mg, more particularly from 100 to 300 mg of the RIPK-1 inhibitor.

In some embodiments, the invention concerns the composition for use as defined above, wherein said composition further comprises a pharmaceutically acceptable excipient.

In the present invention, "pharmaceutically acceptable" is intended to mean that which is useful in the preparation of a pharmaceutical composition, generally safe, nontoxic and neither biologically nor otherwise undesirable and acceptable for both veterinary and human pharmaceutical use.

By "pharmaceutically acceptable excipient" is meant, according to the invention, a non-pharmaceutically active additive used in the manufacture of a pharmaceutical composition, which allows the pharmaceutically active ingredient to be manufactured into a pharmaceutical formulation or a galenic formulation providing the necessary bioavailability of the medicament to the patient upon the administration of the pharmaceutical composition. The excipient is preferably compatible with the other ingredients of the composition and produces no adverse effect, allergic reaction or other undesirable reaction when it is administered to a human or an animal.

The RIPK-1 inhibitor or the composition comprising the same can be administered (either alone or in combination with other pharmaceuticals) in any conventional manner by any route where they are active. For example, administration can be, but is not limited to, by enteral route, in particular by oral or rectal administration, or by parenteral route, in particular by injection. The selection of the specific route of administration and the dose regimen is to be adjusted or titrated by the clinician according to methods known to the clinician to obtain the desired clinical response.

The RIPK-1 inhibitor or the composition comprising the same may be in any forms allowing its administration to the patient, for example a solution, a food, a beverage, a pill, a tablet, a syrup, a patch, a gum, a cream, a gel, a lotion, an ointment, a powder, a capsule, a vial, a suppository, etc ...

In preferred embodiments, the RIPK-1 inhibitor or the composition comprising the same is in a form allowing an oral administration.

The amount administered depends on the RIPK-1 inhibitor formulation, the severity of the condition being treated, the host, the route of administration, etc. It is generally empirically determined and can be adapted in routine trials.

Generally, the quantity of active RIPK-1 inhibitor in a unit dose of preparation may be varied or adjusted from about 1 to 1000 mg, according to the particular application.

In an embodiment, the RIPK-1 inhibitor or the composition comprising the same is formulated into unit dose forms from 1 to 1000 mg, in particular from 50 to 500 mg, more particularly from 100 to 300 mg of said RIPK-1 inhibitor.

In an embodiment, the RIPK-1 inhibitor or the composition comprising the same is formulated into unit dose forms of 1, 2, 3, 4, 5, 6, 7, 8, 9, 10, 20, 30, 40, 50, 60, 70, 80, 90, 100, 150, 200, 250, 300, 350, 400, 450, 500, 550, 600, 650, 700, 750, 800, 850, 900, 950 or 1000 mg of said RIPK-1 inhibitor.

In an embodiment, the RIPK-1 inhibitor or the composition comprising the same is administered to a patient at a regimen of 1 to 1 000 mg of RIPK-1 inhibitor per kg, in particular from 50 to 500 mg per kg, more particularly from 100 to 300 mg per kg.

The RIPK-1 inhibitor or the composition comprising the same can be administered one or more times per day. The regimen can be easily adjusted by the person skilled in the art or the clinician.

In an embodiment, the RIPK-1 inhibitor or the composition comprising the same is administered once daily, twice daily, three times daily or four times daily.

In an embodiment, the RIPK-1 inhibitor or the composition comprising the same is administered once weekly, twice weekly, three times weekly or four times weekly.

Any of the treatment regimens according to the invention can be repeated any number of times. For example, the treatment regimen can be repeated once, twice, 3, 4, 5, 6, 7, 8, 9, 10, 11, 12, 13, 14, 15, 16, 17, 18, 19, 20, or any other number of times.

In an embodiment, the RIPK-1 inhibitor or the composition comprising the same is administered during a meal, preferably in the form of a food or a beverage.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURES

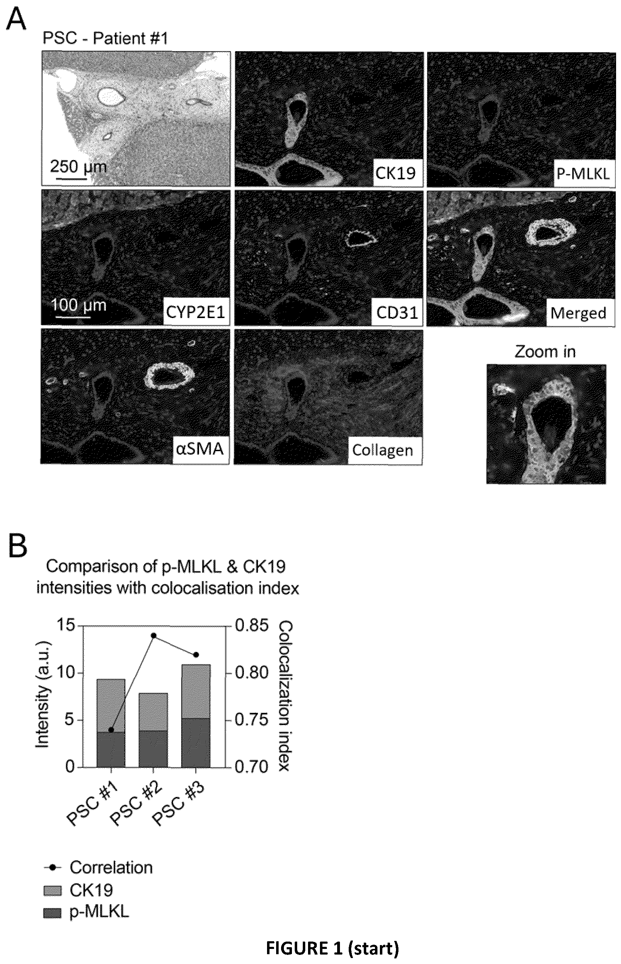

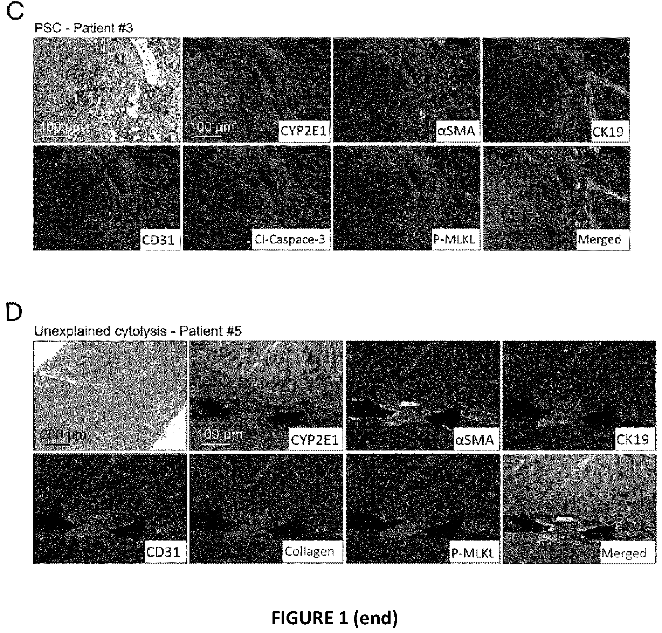

**Figure 1****. Necroptosis activation in PSC is preferentially cholangiocyte-specific, while apoptosis is parenchymal. (A)** Representative multiplex immunofluorescence imaging (COMET platform) from liver biopsy of patient #1 with PSC, stained for CYP2E1 (hepatocytes), α-SMA (smooth muscle cells/myofibroblasts), CK19 (cholangiocytes), CD31 (endothelial cells), collagen (extracellular matrix), and p-MLKL (necroptosis activation). A merged composite image and corresponding hematoxylin/eosin (H&E) staining of the analyzed region are also shown. Inset displays magnified view illustrating strong colocalization between CK19-positive cholangiocytes and p-MLKL staining. **(B)** Quantification of CK19 and p-MLKL staining intensities across liver biopsies from three PSC patients (#1, #2, #3). The secondary Y-axis shows the colocalization index between p-MLKL and CK19, demonstrating a positive correlation indicative of selective necroptosis activation in cholangiocytes. **(C)** Additional representative multiplex imaging from PSC patient #3, stained for CYP2E1, CD31, cleaved caspase-3 (cl-Caspase-3, apoptosis marker), α-SMA, p-MLKL, CK19, and corresponding merged image, highlighting spatial separation between apoptotic and necroptotic cell death signals. Note that the H&E image is from the same biopsy but does not precisely match the COMET-imaged region; however, it remains representative of the tissue's overall morphological features. **(D)** Multiplex immunofluorescence imaging from a patient (#5) presenting unexplained cytolysis without histologically apparent parenchymal lesions, stained for CYP2E1, α-SMA, CK19, CD31, collagen, p-MLKL, with corresponding merged composite image and H&E staining, showing limited cholangiocyte-specific p-MLKL positivity compared to PSC biopsies.

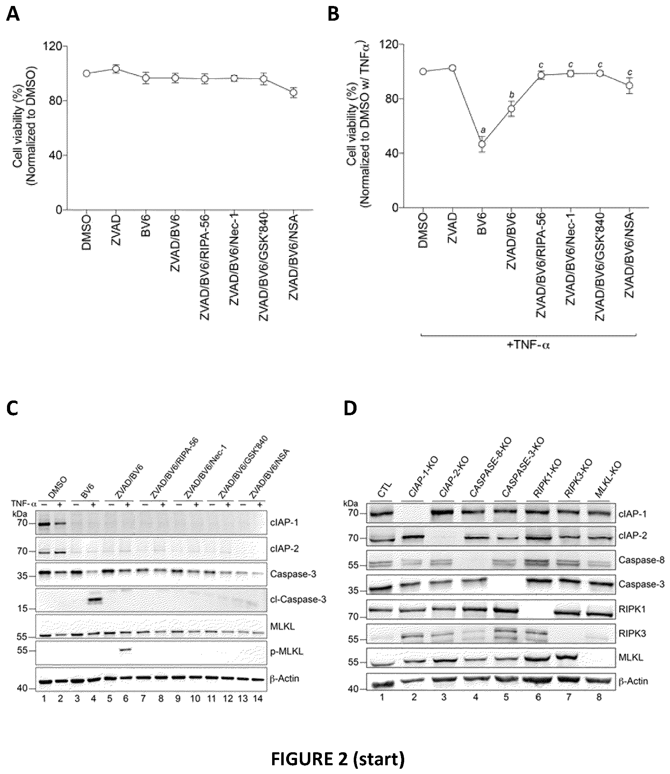

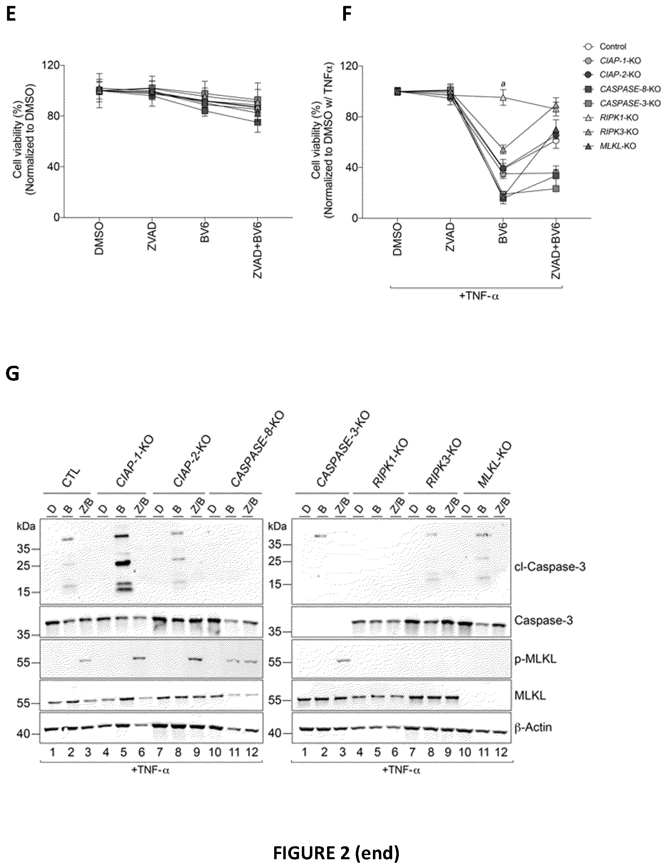

**Figure 2****. Human H69 cholangiocytes undergo both apoptosis and necroptosis upon TNF-α and IAP inhibition. (A)** Viability (% normalized to DMSO control without TNF-α) of H69 cells treated without TNF-α under indicated conditions: DMSO (vehicle), ZVAD, BV6, ZVAD/BV6, ZVAD/BV6/RIPA-56, ZVAD/BV6/Nec-1, ZVAD/BV6/GSK'840, and ZVAD/BV6/NSA. **(B)** Viability (% normalized to DMSO control with TNF-α) of H69 cells treated with TNF-α under the same conditions as in (A). **(C)** Western blot analysis of cleaved caspase-3 (cl-Caspase-3) and phosphorylated MLKL (p-MLKL) in H69 cells treated with TNF-α and indicated inhibitors: DMSO, BV6, ZVAD/BV6, ZVAD/BV6/RIPA-56, ZVAD/BV6/Nec-1, ZVAD/BV6/GSK'840, ZVAD/BV6/NSA. β-actin served as loading control. **(D)** Western blot analysis confirming CRISPR/Cas9-mediated knockout (KO) of clAP-1, clAP-2, Caspase-8, Caspase-3, RIPK1, RIPK3, MLKL, and loading control β-actin. **(E)** Viability (% normalized to DMSO without -α) of CRISPR/Cas9 KO cell lines treated with DMSO, ZVAD, BV6, or ZVAD/BV6. **(F)** Viability (% normalized to DMSO with TNF-α) of CRISPR/Cas9 KO cell lines treated with TNF-α under the same conditions as in (E). **(G)** Western blot analysis of cleaved caspase-3 (cl-Caspase-3) and phosphorylated MLKL (p-MLKL) in KO cells treated with DMSO (D), BV6 (B), or ZVAD/BV6 (Z/V) in the presence of TNF-α. β-actin served as loading control. Data shown in viability assays represent three independent experiments, each with three replicates. Values with different superscripts indicate statistically significant differences (ANOVA, p < 0.05): "a" indicates a significant difference compared with all other groups; "b" indicates a significant difference compared with all other groups; "c" indicates a significant difference compared with BV6 and ZVAD/BV6 groups.

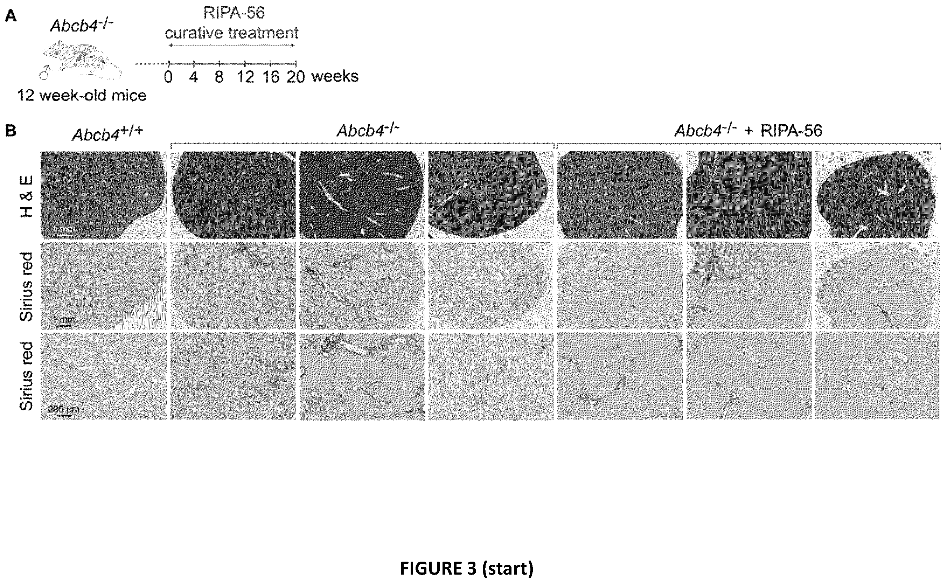

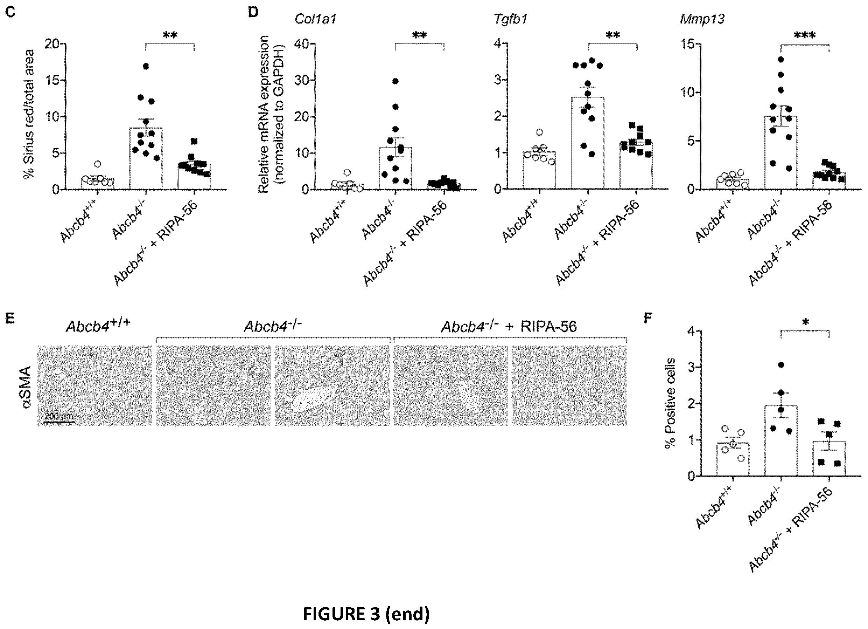

**Figure 3****. Pharmacological RIPK1 inhibition attenuates established biliary fibrosis in Abcb4-deficient mice. (A)** Experimental timeline of curative treatment strategy: twelve-week-old *Abcb4*^{*-*/*-*} mice with established cholestatic liver disease received RIPA-56 treatment for 20 weeks (*n* = 10). Age-matched wild-type (WT, *Abcb4*^{*+*/}*⁺, n* = 7) and untreated *Abcb4*^{*-*/*-*} (KO, *n* = 11) mice served as controls. **(B)** Representative liver histology with Sirius Red and Hematoxylin/Eosin (H&E) staining, untreated KO, and RIPA-56-treated KO mice. **(C)** Quantification of Sirius Red-positive area (% Sirius red/total area) illustrating significant reduction of fibrosis following RIPA-56 treatment. ***p* < 0.01. **(D)** RTqPCR analysis of hepatic mRNA expression for fibrosis-related genes (*Col1a1,* Tgfβ1, and *Mmp13*) from WT, untreated KO, and RIPA-56-treated KO mice. ***p <* 0.01, ****p <* 0.001. **(E)** Immunohistochemical staining for α-smooth muscle actin (α-SMA) from WT, untreated KO, and RIPA-56-treated KO livers, indicating reduced myofibroblast activation following treatment. **(F)** Quantitative analysis of α-SMA-positive cells, confirming significant attenuation of fibrogenic response upon RIPK1 inhibition (*n* = 5 per group). **p* < 0.05.

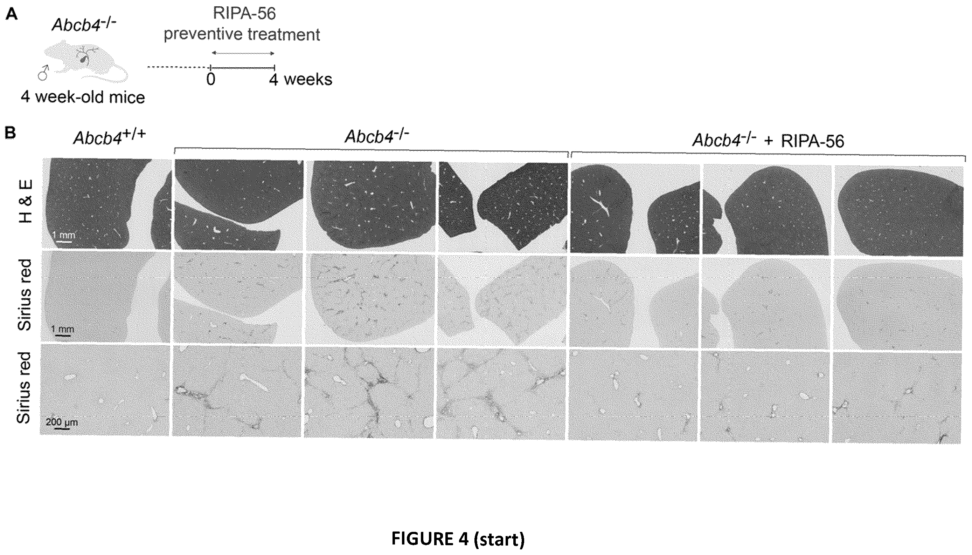

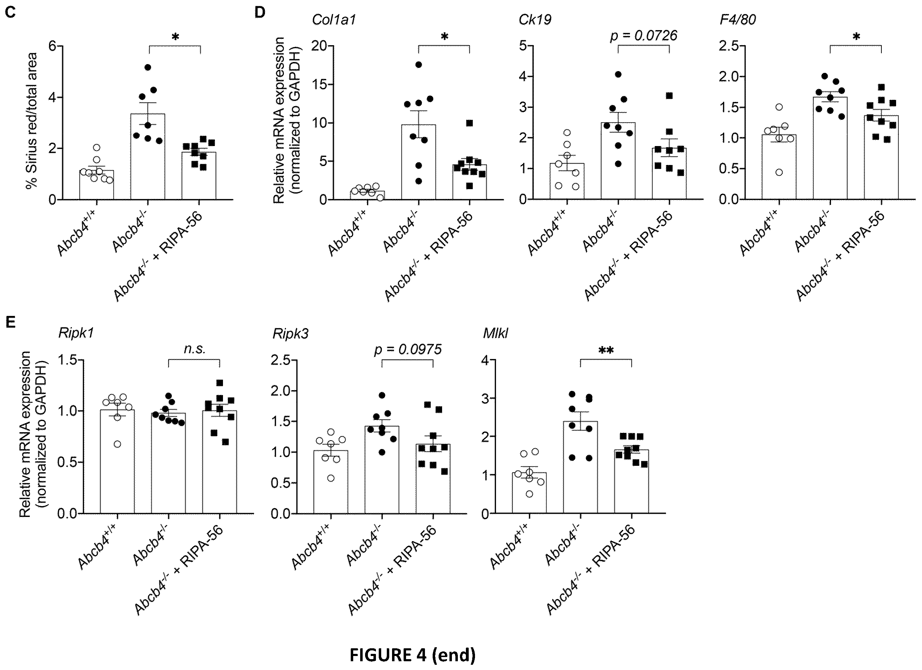

**Figure 4****. Preventive RIPK1 inhibition reduces liver fibrosis and necroptosis mediator expression in male Abcb4-deficient mice. (A)** Experimental treatment scheme: four-week-old *Abcb4*^{*-*/*-*} male mice were treated with RIPA-56 for 4 weeks (preventive approach, initiated prior to significant lesion development, *n* = 9). Age-matched wild-type (WT, *Abcb4*^{*+*/}*⁺, n* = 9) and untreated *Abcb4*^{*-*/*-*} (KO, *n* = 8) littermates served as controls. **(B)** Representative liver histology with Sirius Red and Hematoxylin/Eosin (H&E) staining from WT, untreated KO, and RIPA-56-treated KO mice, illustrating reduced fibrosis following preventive treatment. **(C)** Quantitative image analysis of Sirius Red-positive area, expressed as percentage of total liver area. **p* < 0.05. **(D)** RT-qPCR analysis of hepatic mRNA expression for fibrosis (*Col1a1*), cholangiocyte stress *(Ck19),* and macrophage infiltration *(F4*/*80). *p* < 0.05. **(E)** RT-qPCR analysis of mRNA expression levels for necroptosis mediators *Ripk1, Ripk3,* and *Mlkl. **p <* 0.01.

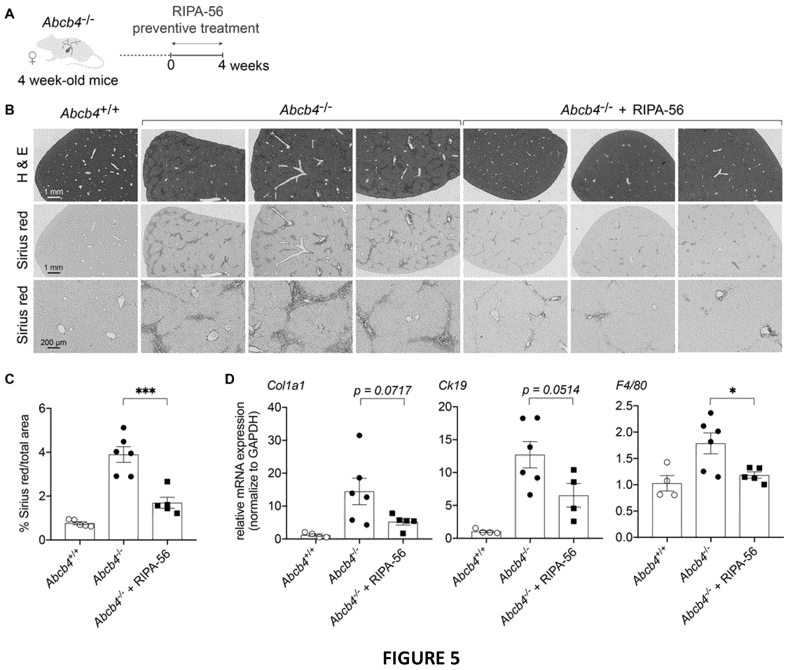

**Figure 5****. Preventive RIPK1 inhibition attenuates liver fibrosis in female Abcb4-deficient mice. (A)** Experimental treatment scheme: four-week-old *Abcb4*^{*-*/*-*} female mice were treated with RIPA-56 for 4 weeks (preventive approach, initiated prior to significant lesion development, *n* = 5). Age-matched wild-type (WT, *Abcb4*^{*+*/}*⁺, n* = 5) and untreated *Abcb4*^{*-*/*-*}(KO, *n* = 6) littermates served as controls. **(B)** Representative liver histology with Hematoxylin/Eosin (H&E) and Sirius Red staining from WT, untreated KO, and RIPA-56-treated KO female mice, showing reduced fibrosis with treatment. **(C)** Quantitative image analysis of Sirius Red-positive area, expressed as percentage of total liver area. ****p <* 0.001. **(D)** RT-qPCR analysis of hepatic mRNA expression for fibrogenic (*Col1a1*), cholangiocyte *(Ck19),* and inflammatory/macrophage (*F4*/*80*) markers. **p* < 0.05.

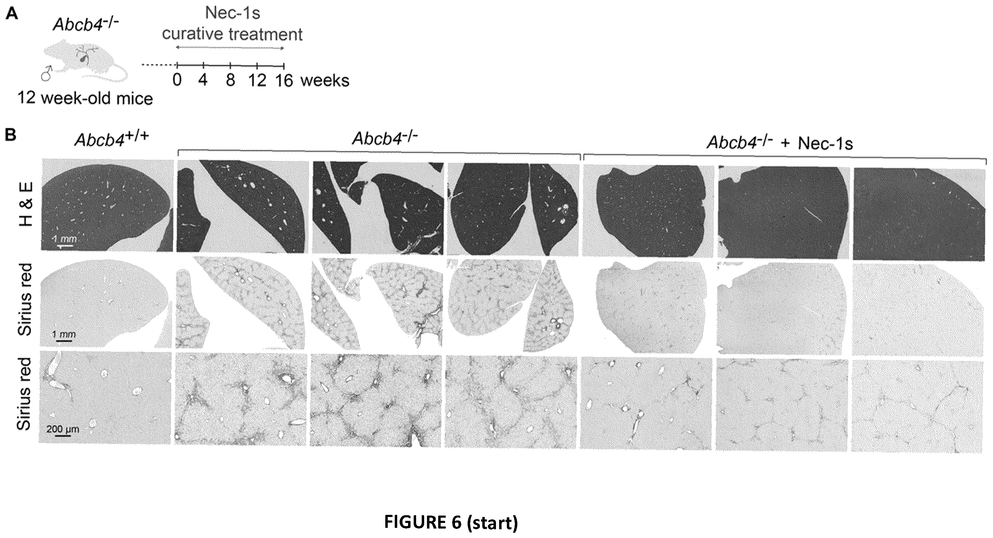

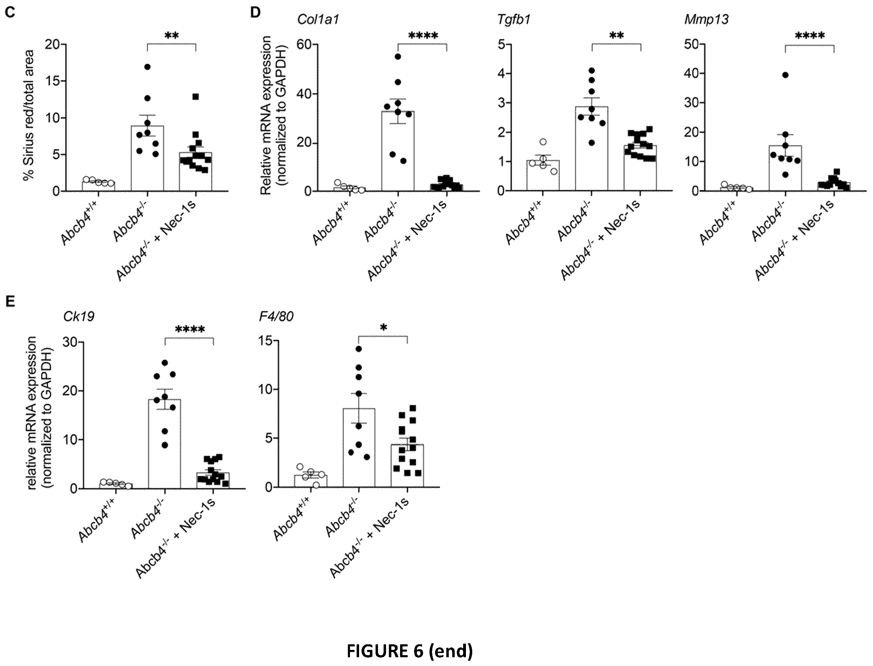

**Figure 6****. Curative RIPK1 inhibition with Nec-1s also attenuates liver fibrosis in *Abcb4-*deficient mice. (A)** Experimental treatment scheme: Twelve-week-old male *Abcb4*^{-/-} mice were treated with Nec-1s for 16 weeks (curative approach initiated after significant lesion development, *n* = 13). Age-matched wild-type (WT, *Abcb4*^{+/+}*, n =* 5) and untreated *Abcb4*^{-/-}(KO, *n* = 8) served as controls **(B)** Representative liver histology showing Hematoxylin/Eosin (H&E) and Sirius Red staining from WT, untreated KO, and Nec-1s-treated KO mice, demonstrating reduced fibrosis upon treatment. **(C)** Quantitative analysis of Sirius Red-positive area, expressed as a percentage of total liver area. ***p <* 0.01, *****p <* 0.0001 **(D-E)** RT-qPCR analysis of hepatic mRNA expression of fibrogenic (*Col1a1*, *Tgfb1,* and *Mmp13*), cholangiocyte *(Ck19),* and inflammatory/macrophage (*F4*/*80*) markers. **p* < 0.05, *****p <* 0.0001.

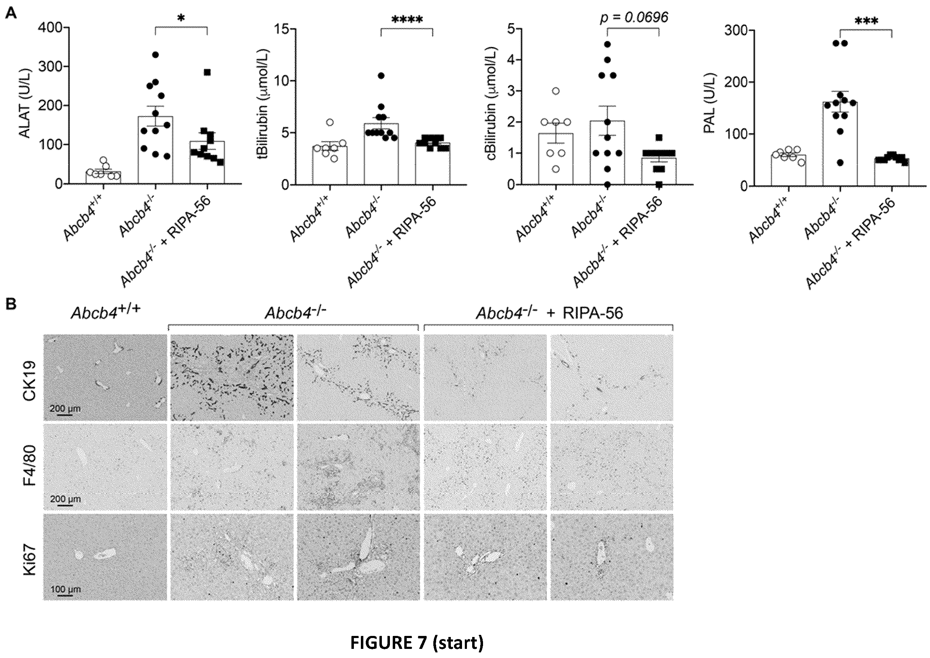

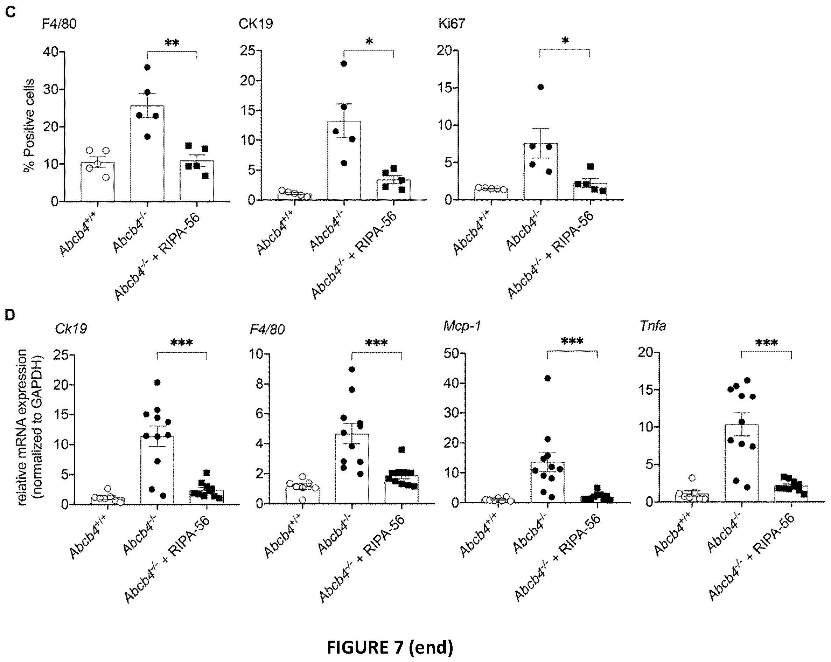

**Figure 7****. RIPK1 blockade improves hepatobiliary damage and reduces inflammatory cell infiltration in Abcb4-deficient mice. (A)** Serum biochemical analysis of alanine aminotransferase (ALAT), total bilirubin (t-Bil), conjugated bilirubin (c-Bil), and alkaline phosphatase (ALP) levels in WT (*Abcb4*^{+/+}*, n* = 7), untreated *Abcb4*^{-/-} (KO, *n* = 11), and RIPA-56-treated *Abcb4*^{*-*/*-*} (KO + RIPA-56, *n* = 10) mice. **p* < 0.05, ***p <* 0.01. **(B)** Representative immunohistochemical staining of CK19 (cholangiocytes), F4/80 (macrophages), and Ki67 (proliferation marker) in liver sections from WT, untreated KO, and RIPA-56-treated KO mice. **(C)** Quantitative analysis of immunohistochemical staining (% Positive cells) for CK19, F4/80, and Ki67, demonstrating reduced cholangiocyte stress, macrophage infiltration, and preserved hepatocyte proliferation following RIPA-56 treatment (*n* = 5 per group). **p <* 0.05, ***p* < 0.01. **(D)** RTqPCR analysis of hepatic mRNA expression for CK19, F4/80, and inflammatory markers MCP-1 and TNF-α in WT, untreated KO, and KO + RIPA-56 mice ***p < 0.001.

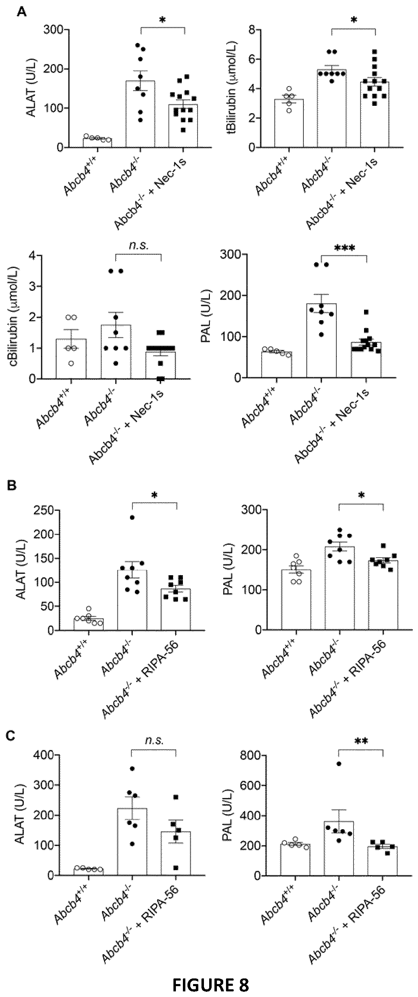

**Figure 8****. Curative Nec-1s and prophylactic RIPA-56 treatments reduce cholestatic injury markers in *Abcb4*^{*-*/*-*} mice. (A)** Serum biochemical analysis of alanine aminotransferase (ALAT), total bilirubin (t-Bil), conjugated bilirubin (c-Bil), and alkaline phosphatase (ALP) levels in WT (*Abcb4*^{*+*/}*⁺, n =* 5), untreated *Abcb4*^{-/-} (KO, *n* = 8), and Nec-1s-treated *Abcb4*^{-/-}mice (n = 13) under a curative regimen. **p* < 0.05, ****p <* 0.001, n.s., non-significant. **(B)** Serum ALAT and ALP levels in WT *(n* = 7), untreated KO (*n* = 8), and prophylactically RIPA-56-treated KO male mice (*n* = 8). **p* < 0.05. **(C)** Serum ALAT and ALP levels in WT (*n* = 5), untreated KO (*n* = 6), and prophylactically RIPA-56-treated KO female mice (*n* = 5). ***p <* 0.01, *n*.*s*., non-significant.

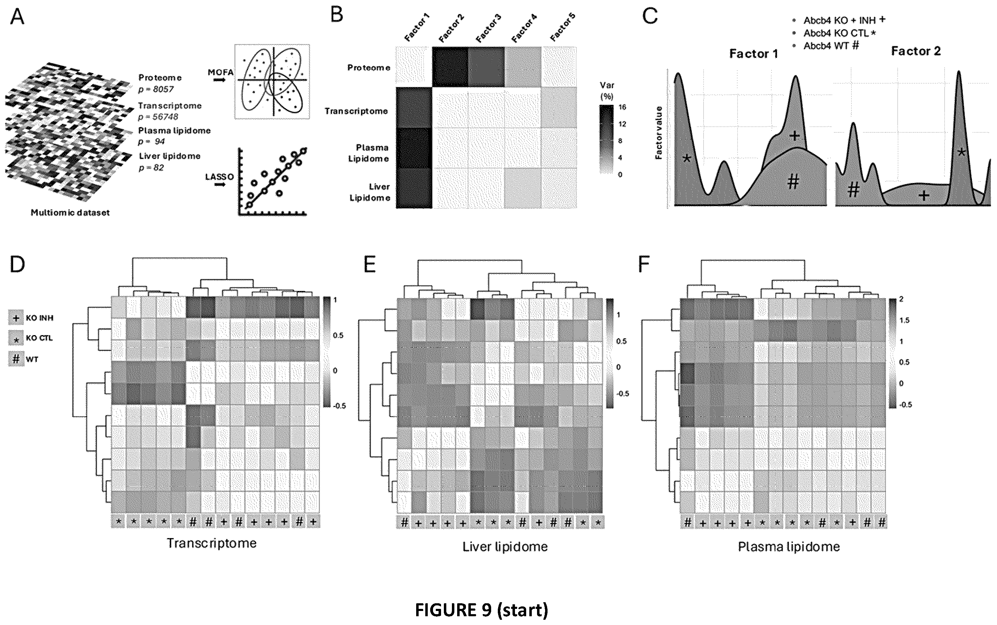

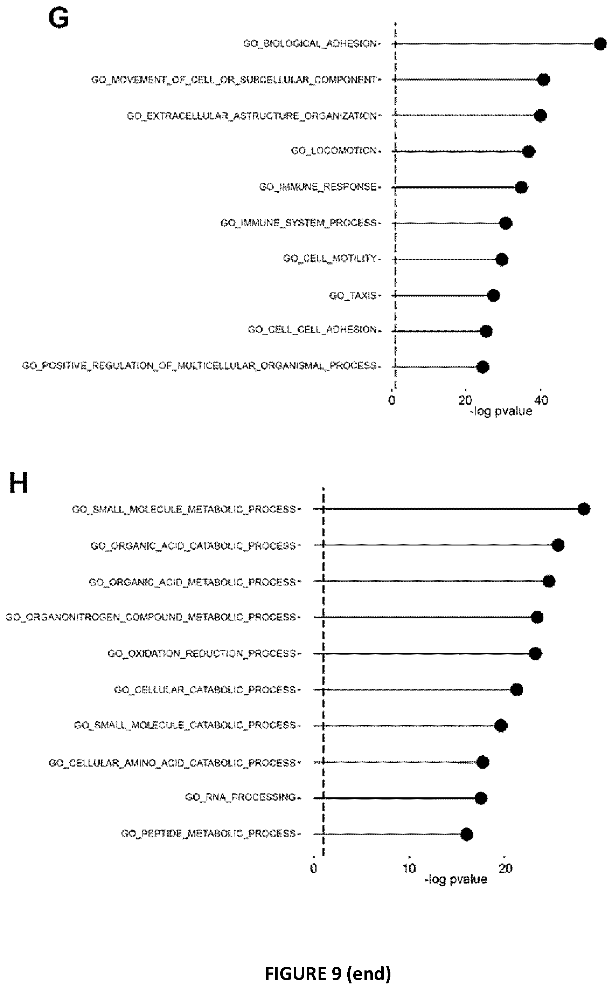

**Figure 9****. RIPK1 inhibition induces multi-omic reprogramming toward WT-like molecular profiles in *Abcb4*-deficient mice. (A)** Overview of the analysis and dataset sizes. **(B)** Multi-Omics Factor Analysis (MOFA) displaying the variance explained (%) by each factor across four omics layers: proteome, transcriptome, plasma lipidome, and liver lipidome. **(C)** Distribution of factor scores across experimental groups (KO INH, KO CTL, WT) for the two main factors explaining the most variance. **(D, E, F)** Hierarchical clustering of the top 10 features from Factor 1 in **(D)** Transcriptome, **(E)** Liver lipidome, and **(F)** Plasma lipidome, with samples colored by experimental condition (KO INH, KO CTL, WT). **(G, H)** Pathway enrichment analysis for Factor 1, which is primarily driven by transcriptomics. Pathways are separated based on downregulated **(G)** vs. upregulated **(H)** loadings and ranked by significance (-log p-value).

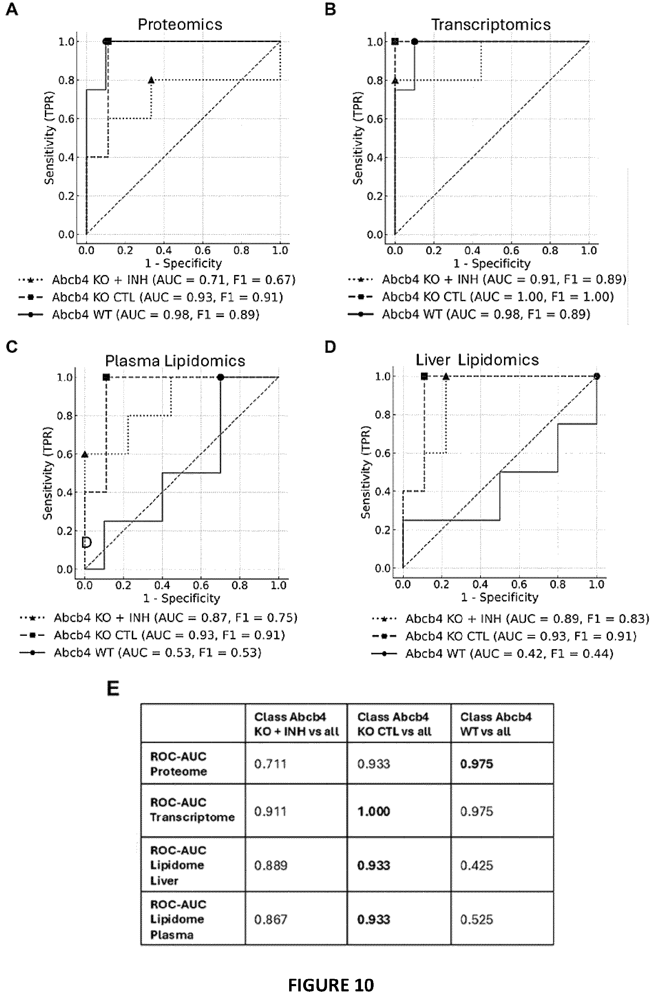

**Figure 10****. Multi-omic molecular signatures accurately discriminate *Abcb4*^{*-*/*-*} mice from RIPA-56-treated mice and WT controls. (A-D)** Receiver operating characteristic (ROC) curves from a supervised multiclass LASSO regression with leave-one-out cross-validation, illustrating the ability of molecular profiles from each omics dataset to distinguish between WT (*Abcb4*^{*+*/*+*}), untreated *Abcb4*^{*-*/*-*} (KO CTL), and RIPA-56-treated *Abcb4*^{*-*/*-*} (KO INH) mice. Classification results for each omics layer are shown separately: **(A)** Proteome, the classification achieved an ROC-AUC of 0.711 for KO INH vs. all, 0.933 for KO CTL vs. all, and 0.975 for WT vs. all, **(B)** Transcriptome the ROC-AUC was 0.911 for KO INH vs. all, 1.000 for KO CTL vs. all, and 0.975 for WT vs. all, **(C)** Plasma Lipidome, the ROC-AUC values were 0.867 for KO INH vs. all, 0.933 for KO CTL vs. all, and 0.525 for WT vs. all and **(D)** Liver Lipidome, the ROC-AUC values were 0.889 for KO INH vs. all, 0.933 for KO CTL vs. all, and 0.425 for WT vs. all. High ROC-AUC scores indicate a strong discriminative ability of the molecular signature for each class. **(E)** A compilation of the ROC-AUCs for the different OMICs across each experimental group is shown.

### EXAMPLES

### MATERIALS & METHODS

### Study design

The therapeutic potential of RIPK1 inhibition was examined both *in vitro* and *in vivo.* For *in vitro* studies, human H69 cholangiocytes were treated with combinations of BV6, TNF-α, and inhibitors of necroptosis pathways, including RIPA-56, Nec-1, and Necrosulfonamid (NSA), to evaluate their effects on cell viability. Cell death pathways were analyzed using viability assays, caspase inhibition (ZVAD), and immunoblotting for necroptotic markers (*e.g.,* p-MLKL). For *in vivo* studies, RIPK1 inhibition was evaluated in the *Abcb4*^{*-*/*-*} mouse model, which recapitulates key features of human PSC, including chronic cholestatic injury, inflammation, and fibrosis in contrast to acute models of liver injury. Male and female mice were randomly assigned to experimental groups, which included RIPA-56-treated, Nec-1s-treated, and untreated cohorts. Both preventive (4 weeks of age) and curative (12 weeks of age) treatment regimens were tested. Liver fibrosis was assessed histologically by Sirius Red staining, while inflammatory markers and cytokine levels were analyzed using RTqPCR and immunohistochemistry. Multi-Omics Factor Analysis (MOFA), an unsupervised dimensionality reduction approach, was used to identify latent factors capturing both shared and dataset-specific variability across multiple omics layers. It was applied to an integrated dataset combining transcriptome, proteome, plasma lipidome, and liver lipidome.

Sample sizes for each experimental group are listed in the figure legends and were determined based on prior studies, sample availability, and published literature. The investigators were not completely blinded during animal treatments or sample collection. However, biochemical, histological, and multi-omic analyses were performed in a blinded manner. All attempts at replication were successful, and no data were excluded during this study. All experiments involving animals were conducted in the SPF animal facility of CRSA (Agreement No. C-75-12-01), according to the European Communities Council Directive (2010/63/UE) for the care and use of animals for experimental procedures and complied with the regulations of the French Ethics Committee of Animal Experiments « Charles Darwin » registered at the « Comité National de Réflexion Ethique sur l'Experimentation Animale » (Ile-de-France, Paris, n°5). All procedures were approved by this committee (n°40428-2023012011546954 v4).

### Multiplex Immunofluorescence

4 µm formalin-fixed paraffin-embedded (FFPE) human liver tissue sections were initially baked overnight at 37°C to soften the paraffin. Slides were manually deparaffinized through three sequential baths of Clearene (Leica Biosystems, Wetzlar, Germany), each lasting 2 minutes. Rehydration was then performed using graded ethanol baths (100%, 90%, 70%, 50%), followed by immersion in distilled water for 2 minutes. Slides were briefly stored in 1X Tris-Buffered Saline (TBS) (Merck, Darmstadt, Germany) prior to antigen retrieval. Antigen retrieval was carried out using the Epredia PT Module with pH 9 Dewax and Heat-Induced Epitope Retrieval (HIER) buffer (Epredia). Slides were immersed into the retrieval solution after a pre-warm cycle at 85°C. Antigen retrieval was performed at 100°C for 1 hour, followed by gradual cooldown to 65°C. Multiplex immunofluorescence staining was conducted using the COMET platform (Lunaphore Technologies, Switzerland). Prior to the staining procedure, the COMET imaging chip (15 mm²) was precisely placed on a predefined region of interest, selected based on a corresponding hematoxylin and eosin (H&E)-stained slide annotated by a pathologist. An initial autofluorescence imaging cycle was conducted, followed by sequential cycles of antibody staining, imaging, and elution. Each staining cycle simultaneously utilized one rabbit and one mouse primary antibody, detected respectively by Alexa Fluor 647-conjugated anti-rabbit and Alexa Fluor 555-conjugated anti-mouse secondary antibodies. After each imaging cycle, antibodies were removed using Elution Buffer (Lunaphore). For image acquisition, an integrated microscope within the COMET system captured images at 20× magnification using DAPI, Cy5, and TRITC lasers. Upon completion of all staining cycles, a fully stacked OME.tif file was automatically generated. Prior to analysis, background subtraction was automatically applied using the Lunaphore COMET Viewer software, and the processed OME.tif images were subsequently exported for further analysis.

### Cell culture

H69 cells, an SV40-transformed (i.e., immortalized) normal human cholangiocyte cell line were grown in Dulbecco's Modified Eagle Medium (DMEM)/F12 (3:1) (Thermo Fisher Scientific, MA, USA) supplemented with 10 % fetal bovine serum (FBS) (Sigma-Aldrich, MI, USA), penicillin (100 IU ml-1), streptomycin (0.1 mg ml-1), 1.8 × 10⁻⁴ M adenine (Sigma-Aldrich), 5 µg/mL insulin (Sigma-Aldrich), 5.5 × 10⁻⁶ M epinephrine (Sigma-Aldrich), 2 × 10⁻⁹ M triiodothyronine (Sigma-Aldrich), 5 µg/mL transferrin (Sigma-Aldrich), 1.64 × 10⁻⁶ M epidermal growth factor (EGF) (Sigma-Aldrich), and 1.1 × 10⁻⁶ M hydrocortisone (Sigma-Aldrich). Cells were maintained at 37°C in a humidified incubator with 5% CO₂ and stimulated with TNF-α (20 ng/µL, PeproTech^{®}, Neuilly-sur-Seine, France) for 8 hrs. H69 cells had undetected mycoplasma contamination (MycoAlertTM PLUS Mycoplasma Detection Kit; Lonza, Bale, Switzerland).

### CRISPR/Cas9-mediated deletion in H69 cholangiocytes

CRISPR/Cas9-mediated gene editing was used to generate targeted gene deletions in H69 cholangiocytes. The pSpCas9(BB)-2A-GFP (PX458) plasmid, a gift from the Zhang Lab (Addgene, MA, USA), was utilized for this purpose. Guide RNAs (gRNAs) were designed using web-based tools such as CRISPOR to avoid off-target sequences, and their efficiency and specificity were verified through resources like SSC and the CRISPR tool from MIT. Following gRNA design, the sequences were cloned into the PX458 plasmid containing the Cas9 nuclease and GFP as a reporter. Transient transfections were performed using TurboFect (Thermo Fisher Scientific), following the manufacturer's instructions. At 48 hours post-transfection, cells were sorted for high GFP positivity by flow cytometry (Cell Sorting Core Facility, Centre de Recherche Saint-Antoine) were transferred into 24-well plates and propagated for subsequent analysis. To minimize off-target effects and avoid the stable integration of Cas9 into the host genome, a transient plasmid transfection approach was employed instead of viral transduction. This strategy not only reduced prolonged Cas9 expression but also mitigated the risk of off-target mutations. Additionally, clonal populations were obtained using limiting dilution following FACS. For each knockout, effects were verified on at least two independent clones to ensure reliability and reproducibility of the results.

### Cell viability assay

H69 cells were treated with the following drugs: Z-VAD-FMK, a pan-capase inhibitor (20 µmol/L, MedChemExpress, Stockholm, Sweden); a clAP1/2 inhibitor, BV6 (10 µmol/L, MedChemExpress); two RIPK1 kinase inhibitors, RIPA-56 (20 µmol/L, MedChemExpress) or Nec-1s (20 µmol/L, MedChemExpress); a MLKL inhibitor, Necrosulfonamide (NSA) (10 µmol/L, MedChemExpress); a kinase RIPK3 inhibitor, GSK'840 (3 µmol/L, MedChemExpress) as indicated in the respective experimental protocols. Cell viability was determined by using CellTiter-Glo Luminescent Cell Viability Assay (Promega, Madison, Wisconsin, U.S.A) according to manufacturer's instruction.

### Western blot analysis

Cells were homogenized in NP-40 lysis buffer to obtain protein lysates. Thirty micrograms of protein extracts were separated by sodium dodecyl sulfate polyacrylamide gel electrophoresis (SDS-PAGE), transferred to a nitrocellulose membrane and analyzed by immunoblotting.

### Animal studies and procedures

The *C57BL*/*6J Abcb4*^{*-*/*-*} mouse strain was maintained through breeding housing *Abcb4*^{*+*/*-*}heterozygous mice on an *C57BL*/*6J* genetic background. Homozygous (^{-/-}) and wild-type (^{+/+}) offspring were genotyped and used for experimental procedure. Mice were housed at CRSA animal facility in a temperature-controlled, specific pathogen-free environment, on a 12-hour light-dark cycle, with free access to a standard chow and water. RIPA-56 and Nec-1s (MedChemExpress) were incorporated in diet by Ssniff spezialdiäten GmbH (Soest, Germany) according to a standardized protocol. Ssniff company is an approved manufacturer of laboratory animal diets and has the license to produce medicated feed for laboratory animals in accordance with Directive 2001/82/EC. The effects of RIPA-56 and Nec-1s were evaluated by incorporating them into the standard chow diet at 250 mg/kg dose as initially described (Li et al., eLife 6, 2017).

### Immunohistochemistry

Paraffin sections were stained with haematoxylin and eosin (H&E), Sirius Red (SR), or incubated with various primary and secondary antibodies. Formalin-(4%) fixed and paraffin-embedded liver tissue sections were incubated in Bond Primary antibody diluent (Leica Biosystems) and staining was performed on a BOND-MAX immunohistochemistry robot (Leica Biosystems) using BOND polymer refine detection solution for 3,3'-Diaminobenzidine (DAB). Anti-F4/80, anti-CK19, anti-αSMA and anti-Ki67 antibodies were used as primary antibody. Image acquisition was performed on a NanoZoomer S360 slide scanner (Hamamatsu Photonics, Hamamatsu, Japan). Stains were quantified densitometrically (area stained per total tissue area) using QuPath software and normalized to total tissue area.

### Real-time quantitative PCR (RTqPCR)

Total RNA was purified from cells using Nucleospin RNAkit (Macherey-Nagel GmbH & Co, Düren, Germany). The quantity and quality of RNA were determined spectroscopically using a nanodrop (Thermo Fisher Scientific). Total RNA (2 µg) was used to synthesize cDNA using the M-MLV reverse transcriptase kit (Thermo Fisher Scientific) according to the manufacturer's protocol. The cDNA samples (20 ng) were used for RT-qPCR in a total volume of 10 µL using SYBR Green Reagent (Roche Diagnostics, Meylan, France) and specific primers, on a LightCycler 96 Roche Instrument. All RT-qPCRs were performed in duplicate. Data were generated and analyzed using the LightCycler 96 software 1.1.0. All values were normalized for the level of *Gapdh* mRNAs.

### RNA sequencing

Total RNA was extracted from liver tissue using the Nucleospin RNA kit (Macherey-Nagel). RNA integrity (RIN) was assessed using Bioanalyzer 2100 system (Agilent Technologies, CA, USA). RNA with a RIN above 9 were used to establish the sequencing libraries. PolyA mRNA selection, cDNA libraries preparation and sequencing were conducted by Novogene Co., LTD (Beijing, China) using the Illumina NOVAseq 6000 platform and 150bp paired-end reads sequencing. Sequencing depth ranged from 56'940'270 to 74'363'506 reads per sample. Raw reads were obtained by CASAVA base recognition. Reads' quality check was examined using FastQC v0.11.9. Clean reads were obtained using Fastp v0.20.1 to remove low-quality reads and read adapters. Clean reads were mapped on the USCS *Mus musculus* mm39 reference genome using HISAT2 v2.0.5. Transcripts' abundance was quantified using featureCounts 1.5.0-p3. The differential expression analysis was performed using the DEseq2 1.20.0 package and EdgeR 3.22.5. Genes with an adjusted P-value <0.05 were further used for analysis. R software and ggplot2 v3.3.4 were used to generate volcano plots and perform the principal component analysis across biological replicates for each condition.

### Proteomics

Tissue lysates were prepared using BeatBox Tissue Kit 24x (PreOmics): samples were homogenized in 5% SDS 50 mM TEAB lysis buffer for 10 minutes applying high BeatBox setting. Three cycles were performed. After heating at 95° for 5 minutes and centrifugation at 14'000 g for 10 minutes, protein dosage was performed using DC Protein Assay Kit (Bio-rad, CA, USA). S-TrapTM micro spin plate (Protifi, Huntington, USA) digestion was performed on 30 µg of cell lysates according to manufacturer's instructions. Briefly, samples were reduced with 20 mM TCEP (Tris(2-carboxyethyl) phosphine hydrochloride) and alkylated with 50 mM CAA (chloracetamide) for 5min at 95°C. Aqueous phosphoric acid was then added to a final concentration of 2.5% following by the addition of S-Trap binding buffer (90% aqueous methanol, 100 mM TEAB, pH 7.1). Mixtures were then loaded on S-Trap plate. Four washes were performed for thorough SDS elimination. Samples were digested with 1.5 µg of trypsin (Promega) at 47°C for 2 hrs. After elution, peptides were vacuum dried and resuspended in 30 µL of 2% ACN, 0.1% formic acid in HPLC-grade water prior to MS analysis. 400 ng of each sample was injected on a nanoElute2 (Bruker Daltonics, Germany) HPLC (high-performance liquid chromatography) system coupled to a timsTOF Pro (Bruker Daltonics, Germany) mass spectrometer. HPLC separation (Solvent A: 0.1% formic acid in water; Solvent B: 0.1% formic acid in acetonitrile) was carried out at 250 nL/min using a packed emitter column (C18, 25 cm×75µm 1.6µm) (lonOptics, Australia) using a 40 min gradient elution (2 to 11% solvent B during 19min; 11 to 16% during 7min; 16% to 25% during 4 min; 25% to 80% for 3 min and finally 80% for 7 min to wash the column). Mass-spectrometric data were acquired using the parallel accumulation serial fragmentation (PASEF) acquisition method in DIA mode. diaPASEF setting were: mass range from 475 to 1000 m/z, mobility ranges from 0.85 to 1.27 V s/cm² 1/k0, cycle time estimate of 0.95 s, mass width of 25 Da and 21 mass steps for cycle. Data analysis was performed using DIA-NN software (version 1.9). A search against the Mus musculus (UniProtKB/Swiss-Prot) database (release July 2024, 17212 entries) was performed using library free workflow. For this purpose, "FASTA digest for library free search/library generation" and "Deep learning spectra, RTs and IMs prediction" options were checked for precursor ion generation. A maximum of 1 trypsin missed cleavages was allowed and the maximum variable modification was set to 2. Carbamidomethylation (Cys) was set as the fixed modification, whereas protein N-terminal methionine excision, methionine oxidation and N-terminal acetylation were set as variable modifications. The peptide length range was set to 7-30 amino acids, precursor charge range 2-4, precursor m/z range 300-1300, and fragment ion m/z range 300-1300. To search the parent mass and fragment ions, accuracy was set to 10 ppm manually. The false discovery rates (FDRs) at the protein and peptide level were set to 1%. Match between runs was allowed. For the quantification strategy, Robust LC (high precision) was used as advised in the software documentation, whereas default settings were kept for the other algorithm parameters. Quantitative matrices were processed with Mass Dynamics 2.0 . In brief, all protein intensities were log2 transformed to perform statistics. For statistical comparison, we set 3 groups, each containing 5 or 6 biological replicates. Then, the data were filtered to keep only proteins with at least 50% valid values in at least one group. Next, the data were imputed to fill missing data points by creating a Gaussian distribution of random numbers with a standard deviation of 30% relative to the standard deviation of the measured values and 1.8 standard deviation downshift of the mean to simulate the distribution of low signal values.

### Lipidomic analysis by LC-MS/MS

Bile acid deuterated internal standards were purchased from Merck/Sigma-Aldrich (CA3Sd4, GCA3Sd4, GLCA3Sd4, LCA3Sd4, TCA3sd4, UDCA3sd4), Steraloids (CAd4, CDCAd4, DCAd4, GCAd4, GCDCAd4, LCAd4), CDN isotopes (GDCAd4, GLCAd4, GUDCAd4, TCAd4) or Eurisotop (βMCAd5, TCDCAd4, TLCAd4). LC/MS grade or UPLC grade solvents were used without further purification and obtained from Merck. Bile acids were extracted from liver and plasma using protein precipitation in acetonitrile as follow. Liver tissues were supplemented with water to a final concentration of 100 mg/ml and homogenized on a Precellys Evolution instrument using the soft program. 30 µl of plasma or 100 µl of homogenized liver (equivalent to 10 mg tissue) were supplemented with deuterated internal standards. Protein precipitation was triggered by the addition of 7 volumes of cold acetonitrile. Mixtures were vortexed for 1 minute, centrifuged at 10'000 g for 1minute and left at 4°C for 1 hr for total precipitation. Mixtures were centrifuged at 20'000 g for 20 min at 4°C and the upper phase collected, dried and resuspended in 50 µl of 50% methanol. A control plasma was extracted in parallel and injected every 10 samples to correct for signal drift. Bile acids were analyzed by LC-MS/MS using a Prominence UFLC and a QTrap 4000 mass spectrometer. Sample (4 µl) was injected into an Ascentis Express C18 column 2.1x150mm. Mobile phases consisted of water and acetonitrile containing 0.1% formic acid. Bile acids were detected using scheduled multiple reaction monitoring (sMRM) in the negative-ion mode. Bile acid concentrations were either expressed as absolute concentration in plasma (nmol/ml) or mass of liver (nmol/g) or as relative values as a percentage of total bile acids.

### Multi-Omics Factor Analysis (MOFA)

To identify latent factors capturing both shared and data set-specific variability across multiple omics layers, the Multi-Omics Factor Analysis (MOFA), an unsupervised dimensionality reduction approach, was employed. MOFA was applied to an integrated dataset encompassing all four omics modalities (transcriptomics, proteomics, plasma lipidomics, and liver lipidomics), and run with the MOFA2 version 1.8.0 package of R. Model hyperparameters were optimized using an expectation-maximization (EM) algorithm, and the optimal number of factors was determined based on variance explained (R²) and model evidence lower bound (ELBO) metrics. The proportion of variance explained by each MOFA factor was calculated across individual omics layers, identifying Factor 1 as the primary contributor to molecular variability. The top 10 features most strongly associated with Factor 1 in each omics dataset were extracted, standardized as z-scores, and hierarchically clustered using Euclidean distance.

### Pathway Enrichment Analysis

To functionally interpret the molecular features associated with MOFA Factor 1 and 2, Gene Ontology (GO) enrichment analysis was conducted on the top-ranked transcriptomic and proteomic features. Genes strongly weighted in Factor 1 or 2 were analyzed using an adapted version of the PCGSE algorithm, with the background set defined as all genes expressed in the RNA-seq dataset or proteomic dataset. Enriched pathways were ranked by p-values, and analyses were performed separately for positively and negatively weighted features contributing to each factor, reflecting distinct biological processes associated with each directionality.

### Supervised Multi-Omic Classification and ROC-AUC Analysis

To quantify the molecular distinctiveness of the experimental groups, a supervised multiclass classification approach using LASSO-regularized logistic regression combined with leave-one-out cross-validation (LOOCV) was implemented. Models were trained separately on each omic dataset (transcriptomics, proteomics, liver lipidomics, and plasma lipidomics) to distinguish between the following groups: KO control (*Abcb4*^{-/-} mice) versus all other samples (WT and RIPA-56-treated *Abcb4*^{-/-} mice), KO RIPA-56-treated (KO INH) versus all other samples (WT and KO), and WT versus all KO samples (KO and KO INH). Receiver operating characteristic-area under the curve (ROC-AUC) scores were computed for each classification scenario to assess how clearly each group's molecular signature could be discriminated. A high ROC-AUC for KO CTL indicated a distinct, disease-associated molecular profile, whereas a lower ROC-AUC for KO INH reflected a shift toward a more WT-like molecular state, consistent with molecular rescue.

### Statistical analysis

GraphPad Prism 8.0 and R v4.2.3 software were used. Statistical significance between two groups *(i.e., Abcb4*^{-/-} *vs. Abcb4*^{-/-} RIPA-56-treated mice) was determined by unpaired two-tailed Student's *t* test. Comparisons involving multiple groups were analyzed using one-way analysis of variance (ANOVA) followed by Tukey's post-hoc multiple comparison test. All data are presented as mean ± SEM, and differences were considered statistically significant at p < 0.05. For omics datasets, all data were log2-transformed when necessary to address heteroskedasticity, followed by z-scoring. This preprocessing step was conducted before performing MOFA, LASSO, and differential expression analyses. *P*-values were calculated using the Mann-Whitney non-parametric test, with multiple hypothesis correction applied *via* the Benjamini-Hochberg procedure. Effect sizes are reported as fold changes.

### EXAMPLE 1- Spatial profiling highlights necroptosis in PSC cholangiocytes

Hypothesizing that necroptosis may contribute to biliary injury, it was first examined whether this form of programmed cell death occurs in clinical PSC specimens. To this end, a high-plex immuno-histochemistry platform that enables sequential staining and imaging on a single formalin-fixed paraffin-embedded (FFPE) slide, allowing detailed spatial profiling of diverse cellular populations, was used. The relevant clinical data for the PSC patients included in this study - such as elevated transaminases and alkaline phosphatase levels compared with standard reference ranges - are shown in **Table 1.**

**Table 1. Clinical characteristics and biochemical parameters of PSC patients and "control" individuals (one with unexplained cytolysis without histological lesions, and one with genetically determined cholestasis due to ABCG5 mutation without biliary lesions) included in this study.**

| **Disease** | **PSC Patients** | | | | **Unexplained Cytolysis** | **Drug-Induced cholestasis** |
|---|---|---|---|---|---|---|
| **Patient Number** | #1 | #2 | #3 | #4 | #5 | #6 |
| **Age (years)** | 29 | 36 | 54 | 39 | 59 | 18 |
| **Gender** | Female | Male | Male | Female | Female | Female |
| **Time from diagnosis (years)** | 1 | 2 | 1 | 1 | 1 | 1 |
| **Inflammatory Bowel Disease (IBD)** | No | Yes | No | Yes | No | No |
| **Ulcerative Colitis (UC)** | | No | | Yes | | |
| **Crohn's Disease (CD)** | | Yes | | No | | |
| **Aspartate Aminotransfe rase (ASAT) (IU/L)** | 55 | 72 | 44 | 66 | 65 | 61 |
| **Alanine Aminotransfe rase (ALAT) (IU/L)** | 72 | 146 | 61 | 130 | 80 | 134 |
| **Gamma Glutamyl transpeptidas e (GGT) (IU/L)** | 168 | 1290 | 238 | 384 | 198 | 10 |
| **Alkaline Phosphase (ALP) (IU/L)** | 143 | 245 | 179 | 221 | 99 | 168 |
| **Total bilirubin (µmol/L)** | 14 | 9 | 17.4 | 15 | 7 | 144 |
| **Conjugated bilirubin (µmol/L)** | 5 | 5 | 8.1 | 4 | 2 | 124 |
| **Liver stiffness (kPa)** | 5.6 | 10.2 | 8.4 | | 7.2 | 4.9 |
| **Fibrosis** | Rare septa (F2) | Numerous septa (F3) | Rare septa (F2) | Portal tract fibrosis (F1) | No fibrosis (F0) | No fibrosis (F0) |
| **Ductular reaction** | Yes | Yes | Yes | Yes | No | No |
| **Ductopenia** | No | No | Yes | No | No | No |
| **Ursodeoxycho lic Acid (UDCA) (mg/kg/d)** | 15.3 | 15.0 | 13.8 | 17.9 | No | No |

A multiplex panel that combines cell-type markers including CK19 (Cholangiocytes), CYP2E1 (Hepatocytes), α-SMA (Vascular smooth muscle cells and myofibroblasts), CD31 (Endothelial cells), CD68 (Macrophages), collagen (Extracellular matrix), phosphorylated-MLKL (p-MLKL) and cleaved-Caspase-3 (cl-Caspase-3), was used to identify major hepatic cell populations. A comprehensive view of cellular phenotypes and their spatial relationships in PSC-affected liver tissues was obtained. Phosphorylated-MLKL staining was primarily localized to cholangiocytes, suggesting a preferential activation of necroptosis in these cells in the liver of PSC patients **(****Figures 1A and 1B****).** p-MLKL staining was occasionally detected in hepatocytes, but at substantially lower frequency and intensity compared with cholangiocytes, consistent with observations in patients with end-stage liver diseases, in whom cholangiocytes are positively stained for p-MLKL, while no signal is detected in hepatocytes or in healthy donor livers. Furthermore, a previous RTqPCR analysis of freshly isolated human hepatocytes and primary cholangiocytes showed higher expression of necroptosis-related genes, including RIPK3 and MLKL, in cholangiocytes than in hepatocytes. Together, these findings further support the increased susceptibility of cholangiocytes to necroptosis under pathological conditions, such as PSC.

Because apoptotic pathways have been shown to broadly contribute to liver injury, cl-Caspase-3 staining in the liver of PSC patients was analyzed. cl-Caspase-3 staining was dispersed across multiple hepatic cell populations-most notably in hepatocytes and occasionally in macrophages-but was absent in cholangiocytes **(****Figure 1C****).** This distribution indicates that apoptosis is a widespread mechanism in the context of PSC but cannot account for the specific injury of biliary epithelial cells **(****Figure 1C****).** To further evaluate whether p-MLKL staining is specific to PSC, the present analysis was expanded to two additional liver specimens: one from a patient with unexplained cytolysis (i.e., elevated transaminases) but no histologically apparent lesions, and another with drug-induced cholestasis but without biliary lesions on histology **(Table 1).** In both cases, p-MLKL was detectable in bile ducts, but at clearly lower signal intensity and in only a minority cholangiocytes **(****Figure 1D****).** By contrast, virtually every duct in PSC tissue showed strong, continuous p-MLKL positivity. These observations support the hypothesis that cholangiocytes have an intrinsic susceptibility to necroptosis, markedly pronounced in PSC. The sporadic p-MLKL-positive cholangiocytes seen in the non-PSC livers raises the possibility of "zombie-like" cells-analogous to senescent cells that activate necroptotic signaling without proceeding to complete cell lysis. Such cells, potentially bearing a pro-inflammatory secretome, may persist within the tissue and maintain a pro-inflammatory microenvironment. Taken together, these findings suggest that necroptosis plays a central role in PSC pathophysiology, particularly within cholangiocytes.

### Example 2 - H69 cholangiocytes are sensitive to both apoptosis and necroptosis

To investigate the balance between cell death mechanisms in cholangiocytes, the H69 cell line, a widely adopted *in vitro* model for human biliary epithelial cells, was used. A previous study reported that the expression of cellular inhibitor of apoptosis proteins (clAP)-1 and - 2 is lower in small and large interlobular bile ducts from cirrhotic-stage PSC patients than in normal liver or metabolic associated steatohepatitis (MASH) patients. It was thus decided to examine whether inhibiting clAPs would affect the viability of H69 cells. Therefore, H69 cells were treated with BV6, an IAP antagonist known to induce auto-ubiquitination and subsequent proteasomal degradation of clAP-1 and clAP-2, in the presence of tumor necrosis factor-α (TNF-α). Notably, BV6 alone, without TNF-α, did not induce significant cell death **(****Figure 2A****).** However, cell viability dropped to ~35% when combining BV6 with TNF-α **(****Figure 2B****).** To determine the contribution of apoptosis, cells were preincubated with Z-VAD-FMK (ZVAD), a pan-caspase inhibitor, which increased cell viability from ~35% to ~65% **(****Figure 2B****),** indicating that apoptosis is a major pathway in BV6/TNF-α-induced cell death in H69 cholangiocytes. Because combination of BV6 and ZVAD can trigger necroptosis, inhibitors that specifically target key mediators of necroptosis, i.e. Necrostatin-1 (Nec-1) and RIPA-56 (both selective RIPK1 kinase inhibitors), GSK'840 (a potent RIPK3 kinase inhibitor), and necrosulfonamide (NSA; an MLKL inhibitor), were next employed. Each of these inhibitors rescued H69 cells from ZVAD/BV6/TNF-α-induced death, suggesting that the partial recovery observed with apoptosis inhibitors can be ascribed to the involvement of necroptosis **(****Figure 2B****).** Western blot analyses confirmed involvement of apoptosis after BV6/TNF-α treatment of H69 cells by showing elevated cl-Capsase-3, while addition of ZVAD to BV6/TNF-α treatment increased phosphorylation of MLKL (p-MLKL), confirming the induction of necroptosis under caspase-inhibited conditions **(****Figure 2C****).** The increase in p-MLKL was prevented by specific necroptosis inhibitors, underscoring the sequential activation of apoptosis followed by necroptosis upon caspase inhibition in H69 cells treated with BV6 and TNF-α **(****Figure 2C****).**

To further delineate the contributions of individual pro-death or pro-survival factors governing BV6/TNF-α-induced cell death, a panel of CRISPR/Cas9 knockout (KO) H69 cells targeting *CIAP-1, CIAP-2, CASPASE-8, CASPASE-3, RIPK1, RIPK3,* and *MLKL* was generated. Western blot analysis confirmed successful knockout of each target and showed that the absence of one protein (e.g., Caspase-8) did not induce obvious compensatory changes in the remaining factors (i.e., clAP1, clAP2, Caspase-3, RIPK1, RIPK3, or MLKL) **(****Figure 2D****).** In addition, each KO line was validated by Sanger sequencing to characterize on-target insertions and deletions (indels). In the absence of TNF-α, all KO lines displayed minimal variability in cell viability under BV6 or BV6/ZVAD treatment compared to control cells **(****Figure 2E****).** The impact of ZVAD alone on cell viability was minimal (±TNF-α) **(****Figure 2F****).** Notably, *CIAP1*-KO and *CIAP2*-KO cholangiocytes showed distinct responses to BV6/TNF-α and BV6/ZVAD/TNF-α. *CIAP2*-KO cells behaved similarly to control, with ~40% survival under BV6/TNF-α alone, which increased to ~70% when ZVAD was added, indicating that ~30% of the cells underwent necroptosis **(****Figure 2F****).** By contrast, *CIAP1*-KO cells exhibited ~35% survival under BV6/TNF-α that remained unchanged with BV6/ZVAD/TNF-α, suggesting that clAP1 may regulate an alternative cell death pathway not rescued by caspase inhibition **(****Figure 2F****).**

Then, *CASPASE-8-KO* and *CASPASE-3-KO* cells were evaluated to clarify the interplay between apoptosis and necroptosis. Both lines displayed low survival rate (<20%) following BV6/TNF-α treatment, indicating a pronounced shift toward necroptosis **(****Figure 2F****).** Notably, co-treatment with ZVAD partially rescued cell viability in *CASPASE-8-KO* (~40% survival) but had minimal impact in *CASPASE-3-KO* **(****Figure 2F****),** suggesting that Caspase-8 deficiency alone cannot prevent BV6/ZVAD/TNF-α-induced death and that additional apoptotic pathways may be triggered secondarily. Further insights emerged when MLKL or RIPK3, the core effectors of necroptosis, were disrupted. In *MLKL-KO* cells, BV6/TNF-α treatment yielded ~15% survival and was only partially rescued by ZVAD (~75% survival), implying the involvement of additional, MLKL- and caspase-independent routes of cell death **(****Figure 2F****).** In *RIPK3*-KO cells, ~50% of cells died under BV6/TNF-α alone, yet combining BV6/TNF-α with ZVAD restored viability nearly to 100%, consistent with literature suggesting that RIPK3 also modulates apoptotic signaling. Crucially, *RIPK1-KO* cells were entirely protected from BV6/TNF-α-induced cell death, highlighting RIPK1 as a pivotal upstream regulator of both apoptotic and necroptotic cascades **(****Figure 2F****).** This regulation presumably involves the formation of the ripoptosome, which depends on RIPK1 kinase activity - as it was confirmed using a RIPK1 kinase inhibitor - and can drive Caspase-8-mediated apoptosis or caspase-independent necroptosis. Consistent with these viability data, Western blot analyses of the KO lines for cleaved cl-Caspase-3 and p-MLKL confirmed that removing RIPK1 prevented both apoptotic and necroptotic signaling in H69 cholangiocytes **(****Figure 2G****).** Specifically, in both control and other KO lines, BV6/TNF-α treatment elevated cl-Caspase-3, whereas BV6/ZVAD/TNF-α increased p-MLKL **(****Figure 2G****).** Neither of these effects was observed in *RIPK1*-KO cells, reinforcing the essential role of RIPK1 in coordinating cell fate decisions under these conditions. Together with the data obtained in PSC patients, these observations suggest that necroptosis may be specifically triggered in cholangiocytes in the setting of PSC.

### Example 3 - RIPK-1 inhibition reduces biliary fibrosis in Abcb4-deficient mice

To assess the therapeutic potential of RIPK1 inhibition in an experimental model of PSC, the *Abcb4*^{*-*/*-*} (also referred as *Mdr2*^{-/-} mice) mouse model, which recapitulates key features of human PSC, including progressive cholestatic injury, biliary inflammation and periductal fibrosis, was employed. In this model, it was tested whether pharmacological blockade of RIPK1 with RIPA-56 could alleviate fibrosis in *Abcb4*^{-/-} mice. RIPA-56, a potent, selective and metabolically stable inhibitor of RIPK1, which was shown to prevent MLKL-mediated cell death in a MASLD model, was first used in a curative protocol targeting *Abcb4*^{-/-} mice with established disease **(****Figure 3A****).** Thus, twelve-week-old male *Abcb4*^{-/-} mice on a normal chow diet (NCD) were treated with RIPA-56 for 20 weeks **(****Figure 3A****).** Age-matched wild-type (WT) and untreated *Abcb4*^{-/-} mice served as controls. Histological examination of untreated *Abcb4*^{-/-} livers revealed extensive periductal ECM deposition and dense collagenous bands extending into the parenchyma, consistent with the classical PSC-like phenotype **(****Figure 3B****).** In contrast, RIPA-56-treated *Abcb4*^{-/-} mice showed markedly reduced collagen accumulation, nearing WT levels, as demonstrated by Sirius Red staining **(****Figure 3B****)** and quantitative image analysis **(****Figure 3C****).** At the molecular level, RTqPCR analysis revealed that fibrogenic genes such as *Col1a1, Tgf61* and *Mmp13* were significantly elevated in untreated *Abcb4*^{*-*/*-*} mice, reflecting ongoing ECM-producing myofibroblast activation **(****Figure 3D****).** RIPA-56 treatment substantially lowered these transcripts, restoring their expression toward WT values **(****Figure 3D****).** Immunohistochemistry (IHC) for α-SMA corroborated these findings, revealing fewer α-SMA-positive area in RIPA-56-treated *Abcb4*^{*-*/*-*} livers compared to untreated counterparts **(****Figures 3****, E and F).** Collectively, these findings show that pharmacological RIPK1 inhibition effectively attenuates established biliary fibrosis in *Abcb4*^{*-*/*-*} mice.

To further evaluate the potential of RIPK1 inhibition, a preventive strategy was next employed. Male *Abcb4*^{*-*/*-*} mice received RIPA-56 starting at 4 weeks of age, before significant disease onset, and were sacrificed at 8 weeks **(****Figure 4A****).** After one month of treatment, RIPA-56 significantly reduced fibrosis, as evidenced by histological analysis and RTqPCR measurements of fibrogenic genes **(****Figures 4B** **and** **4D****).** Notably, RTqPCR analysis also revealed a pronounced decrease in *Mlkl* expression, a trend toward reduction in *Ripk3,* and no significant change in *Ripk1* **(****Figure 4E****).** These findings indicate that RIPA-56, by inhibiting the kinase activity of RIPK1, modulates the downstream expression of necroptosis mediators, consistent with prior observations in different experimental rodent models. Together, these findings suggest that early intervention with RIPA-56 can mitigate the initial progression of PSC-like pathology. It was also examined whether RIPK1 inhibition exerts similar effects across sexes by analyzing a cohort of female *Abcb4*^{*-*/*-*} mice. RIPA-56-treated females exhibited reduced fibrosis, as shown by histological staining and decreased fibrogenic gene expression compared to untreated *Abcb4*^{-/-} mice **(****Figure 5****),** indicating that RIPK1 inhibition is efficacious regardless of sex.

To consolidate these observations of a direct impact of RIPK1 targeting on PSC-like features, Necrostatin-2 racemate (Nec-1s), another potent and specific RIPK1 inhibitor lacking the IDO-targeting effect, was tested under similar curative conditions **(****Figure 6****).** Nec-1s treatment significantly decreased fibrogenic gene expression while concurrently reducing cholangiocyte staining *(Ck19)* and macrophage infiltration (*F4*/*80*) toward WT levels, mirroring the protective effects observed with RIPA-56 **(****Figure 6****).** Together, these results demonstrate that RIPK1 kinase inhibition effectively reduces fibrosis in the *Abcb4*^{*-*/*-*}mouse model under both curative and preventive treatment regimens. This therapeutic effect is observed in both male and female mice underscoring the promise of RIPK1 as a potential therapeutic target for PSC.

### Example 4 - RIPK-1 inhibition reduces cholestatic injury and inflammation in Abcb4-deficient mice

Untreated *Abcb4*^{-/-} mice displayed significantly elevated serum levels of alanine aminotransferase (ALAT), total and conjugated bilirubin, and alkaline phosphatase (ALP), indicative of marked hepatobiliary damage **(****Figure 7A****).** Treatment with RIPA-56 lowered all parameters, bringing them closer to WT levels **(****Figure 7A****).** Nec-1s administration likewise diminished cholestatic injury markers. Additionally, prophylactic RIPA-56 treatment significantly reduced ALAT and ALP levels in male *Abcb4*^{-/-} mice, while in female mice only ALP was significantly decreased **(****Figure 8****).** Notably, higher baseline injury marker levels suggest a potentially more severe disease phenotype in female mice, suggesting that a longer treatment duration may be necessary to achieve comprehensive therapeutic effects, including normalization of transaminases **(****Figure 8****).** At the tissue level, immunohistochemistry for CK19 revealed diminished staining in RIPA-56-treated mice, suggesting a lower rate of ductular reaction and a more quiescent biliary epithelium **(****Figure 7B****).** F4/80 staining showed fewer infiltrating macrophages, and Ki-67 indicated that hepatocyte and cholangiocyte proliferation remained at levels comparable to WT under RIPA-56 treatment **(****Figure 7B****).** Quantitative image analysis of these IHC findings corroborated reduced CK19 and F4/80 signals, as well as normal Ki-67 positivity relative to WT mice **(****Figure 7C****).** Consistent with these histological observations, RTqPCR analyses demonstrated significantly lower expression of *Ck19* and *F4*/*80* in RIPA-56-treated mice, along with marked reductions in pro-inflammatory mediators such as monocyte chemoattractant protein-1 (*Mcp-1*) and *Tnfa* **(****Figure 7D****).** Altogether, these data demonstrate that pharmacological RIPK1 inhibition not only attenuates fibrogenic pathways but also alleviates cholestatic injury and suppresses inflammation. Notably, the reduced CK19 expression likely reflects a diminished ductular reaction, together with low proliferation rates in both cholangiocytes and hepatocytes, further underscoring the beneficial effects of RIPK1 inhibition on biliary injury. Collectively, these data show that RIPK1 inhibition broadly mitigates key pathological features of PSC, underscoring the therapeutic potential of targeting necroptosis to restore biliary epithelial homeostasis and limit chronic inflammatory injury in cholestatic liver disease.

### Example 5 - Integrated multi-omics reveals therapeutic reprogramming toward WT-like molecular profiles by RIPK-1 inhibition

To further elucidate the molecular mechanisms underlying the therapeutic effects of RIPK1 inhibition in PSC, a multi-omics factor analysis (MOFA) integrating transcriptomic (RNA-sequencing), proteomic, plasma lipidomic and liver lipidomic datasets was performed **(****Figure 9A****).** This dimensionality reduction approach identified latent factors capturing shared and dataset-specific variability across experimental conditions, specifically comparing RIPA-56-treated and untreated *Abcb4*^{-/-} mice to WT controls. The MOFA variance contribution plot illustrates the proportion of variance explained by each factor across the different omics layers **(****Figures 9B and 9C****).** Factor 1 emerged as the dominant driver of variation, explaining substantial variance across a combination of transcriptomics (~14%), plasma lipidomics (~16%), and liver lipidomics (~12%), with minimal contribution from proteomics **(****Figures 9B and 9C****).** Conversely, Factor 2 accounted for most of the proteomic variation (~16%), suggesting a distinct adaptive response at the protein level following RIPA-56 treatment **(****Figures 9B and 9C****).** The remaining factors contributed minimally to overall variance (<10%), making them less relevant for further analysis **(****Figure 9B****).** Hierarchical clustering analysis of the top 10 features contributing to Factor 1 across transcriptomic **(****Figure 9D****),** liver lipidomic **(****Figure 9E****),** and plasma lipidomic **(****Figure 9F****)** datasets revealed distinct molecular signatures differentiating experimental groups, prominently separating untreated *Abcb4*^{-/-} mice from both WT and RIPA-56-treated *Abcb4*^{*-*/*-*} mice. Pathway enrichment analysis of genes highly weighted in Factor 1 indicated significant enrichment in processes related to biological adhesion, immune response, extracellular structure organization, cell motility, small molecule metabolism, oxidation-reduction, and peptide metabolism, consistent with inflammatory and fibrotic pathways implicated in PSC **(****Figures 9G** **and** **H****).**

These pathways were further investigated by performing detailed differential gene expression analyses across each enriched biological process, comparing untreated *Abcb4*^{*-*/*-*}(KO_CTL) mice to WT controls and RIPA-56-treated *Abcb4*^{-/-} (KO_INH). To visualize the extent of RIPK1 inhibition-induced reprogramming, eight key pathways - oxidation-reduction process, peptide metabolic process, extracellular structure organization, biological adhesion, immune response, immune system process, locomotion and cell motility - were selected and boxplots for the six genes that were most significantly differentially expressed between KO_CTL and KO_INH were generated. These plots reveal statistically significant shifts in gene expression toward WT-like levels, underscoring the robust therapeutic impact of RIPK1 inhibition in mitigating PSC-associated molecular alterations.

To assess how well molecular profiles differentiate the experimental groups, a supervised multi-omic classification using LASSO regression with leave-one-out cross-validation, an approach well-suited for selecting predictive and interpretable molecular signatures was performed. Across all omic layers, untreated *Abcb4*^{*-*/*-*} mice exhibited highly distinct molecular profiles, achieving robust classification accuracy (ROC-AUC ~1.0), especially evident in transcriptomic **(****Figures 10A and 10B****)** and lipidomic **(****Figures 10C and 10D****)** datasets. In contrast, RIPA-56-treated *Abcb4*^{*-*/*-*} mice demonstrated intermediate classification performance (ROC-AUC ~0.71-0.91), consistent with a partial molecular rescue toward WT-like signatures **(****Figures 10A-D****).** Specifically, proteomic data showed moderate predictive power (ROC-AUC = 0.71) for RIPA-56-treated *Abcb4*^{*-*/*-*} mice, suggesting an adaptive rather than fully normalized protein state following treatment **(****Figure 10A****).** Lipidomic datasets similarly revealed lower ROC-AUC scores for treated RIPA-56-treated *Abcb4*^{*-*/*-*} mice compared to untreated *Abcb4*^{-/-} counterparts **(****Figures 10C and D****),** reinforcing the notion that RIPK1 inhibition substantially mitigates disease-driven molecular alterations, although not fully reverting to a healthy WT baseline. Differential analysis of each omic dataset reveals distinct expression profiles, with highly significant differences distinguishing untreated *Abcb4*^{*-*/*-*} mice from other conditions, even after multiple hypothesis testing correction. Collectively, these integrated omics analyses underscore the multifaceted therapeutic effects of RIPK1 inhibition and highlight specific metabolic and inflammatory pathways as critical targets in PSC pathogenesis and treatment.

## Claims

1. A receptor-interacting serine/threonine-protein kinase 1 (RIPK-1) inhibitor or a composition comprising the same for use in the prevention and/or treatment of a cholestatic liver disease.

2. The RIPK-1 inhibitor or the composition comprising the same for use according to claim 1, wherein the cholestatic liver disease is a chronic cholestatic liver disease.

3. The RIPK-1 inhibitor or the composition comprising the same for use according to claim 2, wherein the chronic cholestatic liver disease is a cholangitis, preferably a fibrosing cholangitis.

4. The RIPK-1 inhibitor or the composition comprising the same for use according to claim 3, wherein the cholangitis is a fibrosing cholangitis selected from the group comprising primary sclerosing cholangitis (PSC), primary biliary cholangitis (PBC), IgG4-related cholangitis (IRC), secondary cholangitis induced by immune checkpoint inhibitors, ischemic cholangitis, drug-induced cholangitis (e.g. ketamine), Alagille syndrome, biliary atresia and genetic cholangitis such as Progressive Familial Intrahepatic Cholestasis (PFIC), preferably PSC.

5. The RIPK-1 inhibitor or the composition comprising the same for use according to any one of claims 1 to 4, wherein the RIPK-1 inhibitor is a type I kinase inhibitor, a type II kinase inhibitor or a type III kinase inhibitor.

6. The RIPK-1 inhibitor or the composition comprising the same for use according to any one of claims 1 to 5, wherein the RIPK-1 inhibitor is selected from the group comprising N-benzyl-N-hydroxy-2,2-dimethylbutanamide (RIPA-56), necrostatin-1 stable (5-((7-chloro-1H-indol-3-yl)methyl)-3-methyl-2,4-imidazolidinedione), necrostatin-1 (5-(1H-indol-3-ylmethyl)-3-methyl-2-thioxo-4-imidazolidinone), necrostatin-2 (5-[(7-chloro-1H-indol-3-yl)methyl]-3-methyl-2,4-imidazolidinedione), necrostatin-3 (3-phenyl-3,3a,4,5-tetrahydro-2H-benz[g]indazole, 3-phenyl-2,3,3a,4-tetrahydro[i]benzopyrano[4,3-c]pyrazole, 3-phenyl-2,3,3a,4-tetrahydro[i]benzothiopyrano[4,3-c]pyrazole, 5,5-dioxo-3-phenyl-2,3,3a,4-tetrahydro[1]benzothiopyrano[4,3-c]pyrazole]), necrostatin-4 ((S)-N-(1-[2-chloro-6-fluorophenyl]ethyl)-5-cyano-1-methyl-1H-pyrrole-2-carboxamide), necrostatin-5 (2-[[3,4,5,6,7,8-hexahydro-3-(4-methoxyphenyl)-4-oxo[1]benzothieno[2,3-d]pyrimidin-2-yl]thio]-acetonitrile), necrostatin-7 (5-((3-(4-fluorophenyl)-1H-pyrazol-4-yl)methylene)-2-imino-3-(thiazol-2-yl)thiazolidin-4-one), necrostatin-21, (S)-5-benzyl-N-(7,9difluoro-2-oxo-2,3,4,5-tetrahydro-1H-benzo[b]azepin-3-yl)1H-1,2,4-triazole-3-carboxamide (GSK3145095), DNL747 (SAR443060), (S)-N-(5-methyl-4-oxo-2,3,4,5-tetrahydrobenzo[b][1,4]oxazepin-3-yl)-4,5,6,7-tetrahydro-1H-indazole-3-carboxamide, (S)-*N*-(5-methyl-4-oxo-2,3,4,5-tetrahydrobenzo[b][1,4]oxazepin-3-yl)-4H-1,2,4-triazole-3-carboxamide, (R)-5-methyl-*N*-((S)-5-methyl-4-oxo-2,3,4,5-tetrahydrobenzo[b][1,4]oxazepin-3-yl)-4,5,6,7-tetrahydro-1H-indazole-3-carboxamide, (R)-5-methyl-N-((S)-5-methyl-4-oxo-2,3,4,5-tetrahydrobenzo[b][1,4]oxazepin-3-yl)-4,5,6,7-tetrahydro-1H-indazole-3-carboxamide, (S)-6,6-dimethyl-N-(5-methyl-4-oxo-2,3,4,5- tetrahydrobenzo[b][1,4]oxazepin-3-yl)-4,5,6,7-tetrahydro-1H-indazole-3-carboxamide, (S)-N-(5-methyl-4-oxo-2,3,4,5-tetrahydrobenzo[b][1,4]oxazepin-3-yl)-i',4',5',7'-tetrahydrospiro[cyclopropane-1,6'-indazole]-3'-carboxamide, N-((S)-5-methyl-4-oxo-2,3,4,5- tetrahydrobenzo[b][1,4]oxazepin-3-yl)-1,4,4a,5,5a,6-hexahydrocyclopropa[f]indazole-3-carboxamide, N-((S)-5-methyl-4-oxo-2,3,4,5-tetrahydrobenzo[b][1,4]oxazepin-3-yl)-3,4,5,5a,6,6a-hexahydrocyclopropa[e]indazole-1-carboxamide, (S)-5,5-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (R)-5-(tert-butyl)-N-((S)-5-methyl-4-oxo-2,3,4,5-tetrahydrobenzo[b][1,4]oxazepin-3-yl)-4,5,6,7-tetrahydro-1H-indazole-3-carboxamide, N-((S)-5-methyl-4-oxo-2,3,4,5-tetrahydrobenzo[b][1,4]oxazepin-3-yl)-5-phenyl-4,5,6,7-tetrahydro-1H-indazole-3-carboxamide, N-((S)-5-methyl-4-oxo-2,3,4,5- tetrahydrobenzo[b][1,4]oxazepin-3-yl)-5-(1H-pyrazol-1-yl)-4,5,6,7-tetrahydro-1H-indazole-3- carboxamide, (R)-1-methyl-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (S)-N-(5-methyl-4-oxo-2,3,4,5-tetrahydrobenzo[b][1,4]oxazepin-3-yl)-5-(trifluoromethyl)-1H-indazole-3-carboxamide, (S)-N-(5-methyl-4-oxo-2,3,4,5-tetrahydrobenzo[b][1,4]oxazepin-3-yl)-5-(trifluoromethyl)-1H-pyrazolo[3,4-b]pyridine-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-[1,2,3]triazolo[1,5-a]pyridine-3-carboxamide, (S)-N-(5-methyl-4-oxo-2,3,4,5-tetrahydrobenzo[b][1,4]oxazepin-3-yl)-7-(trifluoromethyl)imidazo[1,5-a]pyridine-1-carboxamide, (S)-N-(5-methyl-4-oxo-2,3,4,5-tetrahydrobenzo[b][1,4]oxazepin-3-yl)-6-(trifluoromethyl)-[1,2,4]triazolo[4,3-b]pyridazine-3-carboxamide, (S)-5-isopropyl-N-(5-methyl-4-oxo-2,3,4,5-tetrahydrobenzo[b][1,4]oxazepin-3-yl)-1H-pyrazolo[3,4-b]pyridine-3-carboxamide, (S)-N-(5-methyl-4-oxo-2,3,4,5-tetrahydrobenzo[b][1,4]oxazepin-3-yl)-5-(perfluoroethyl)-1H-pyrazolo[3,4-b]pyridine-3-carboxamide, N-((S)-5-methyl-4-oxo-2,3,4,5-tetrahydrobenzo[b][1,4]oxazepin-3-yl)-5-(perfluoroethyl)-4,5,6,7-tetrahydro-[1,2,3]triazolo[1,5- a]pyridine-3-carboxamide, (S)-N-(5-methyl-4-oxo-2,3,4,5-tetrahydrobenzo[b][1,4]oxazepin-3-yl)-6-(perfluoroethyl)-[1,2,4]triazolo[4,3-b]pyridazine-3-carboxamide, (S)-5-benzyl-N-(5-methyl-4-oxo-2,3,4,5-tetrahydrobenzo[b][1,4]oxazepin-3-yl)isoxazole-3-carboxamide (GSK481), 1-[(55)-4,5-Dihydro-5-phenyl-1H-pyrazol-1-yl]-2,2-dimethyl-1-propanone, 2,2-Dimethyl-1-(5(S)-phenyl-4,5-dihydro-pyrazol-1-yl)-propan-1-one (GSK963), (S)-5-benzyl-N-(5-methyl-4-oxo-2,3,4,5-tetrahydrobenzo[b][1,4]oxazepin-3-yl)-1H-1,2,4-triazole-3-carboxamide (GSK2982772), 5-(indol-3-ylmethyl)-3-methyl-2-thio-hydantoin), and their derivatives, analogues or salts.

7. The RIPK-1 inhibitor or the composition comprising the same for use according to any one of claims 1 to 6, wherein the RIPK-1 inhibitor is selected from the group comprising RIPA-56, necrostatin-1 stable and necrostatin-1, in particular RIPA-56.

8. The RIPK-1 inhibitor or the composition comprising the same for use according to any one of claims 1 to 7, wherein the RIPK-1 inhibitor or the composition comprising the same is administered by enteral or parenteral route.

9. The RIPK-1 inhibitor or the composition comprising the same for use according to any one of claims 1 to 8, wherein the RIPK-1 inhibitor or the composition comprising the same is in the form of a solution, a food, a beverage, a pill, a tablet, a syrup, a patch, a gum, a cream, a gel, a lotion, an ointment, a powder, a capsule, a vial or a suppository.

10. The RIPK-1 inhibitor or the composition comprising the same for use according to any one of claims 1 to 9, wherein the RIPK-1 inhibitor or the composition comprising the same is formulated into unit dose forms from 1 to 1000 mg, in particular from 50 to 500 mg, more particularly from 100 to 300 mg of the RIPK-1 inhibitor.

11. The composition for use according to any one of claims 1 to 10, wherein said composition further comprises a pharmaceutically acceptable excipient.
